# EUROPEAN PATENT SPECIFICATION

(11) **EP 2 900 803 B1**
(45) Date of publication and mention of the grant of the patent: **06.11.2019**
(21) Application number: 13841509.6
(22) Date of filing: 26.09.2013
(51) Int. Cl.: C12M 3/00, G01N 33/50

(54) **DEVICES AND METHODS FOR SINGLE CELL ANALYSIS**
VORRICHTUNGEN UND VERFAHREN ZUR EINZELZELLENANALYSE
DISPOSITIFS ET MÉTHODES D'ANALYSE MONOCELLULAIRE

(30) Priority: 26.09.2012 US 201261705914 P; 15.03.2013 US 201361789178 P
(43) Date of publication of application: 05.08.2015
(73) Proprietor: Quantumcyte, Inc., San Jose, CA 95125 (US)
(72) Inventor: BUTLER, John, San Jose, CA 95125 (US); CHAUDHURI, Bidhan, San Jose, CA 95111 (US)
(74) Representative: Boult Wade Tennant LLP
(86) International application number: PCT/US2013/062062
(87) International publication number: WO 2014/052685

(56) References cited:
- WO-A1-03/067255
- WO-A2-2007/118208
- US-A1- 2004 166 555
- US-A1- 2010 028 928
- US-A1- 2011 269 645
- US-A1- 2011 281 745
- US-A1- 2012 149 781
- PEPPAS N A ET AL: "Hydrogels in pharmaceutical formulations", EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 3 July 2000 (2000-07-03), pages 27-46, XP004257178, ISSN: 0939-6411, DOI: 10.1016/S0939-6411(00)00090-4
- MIRREN CHARNLEY ET AL: "Integration column: microwell arrays for mammalian cell culture", INTEGRATIVE BIOLOGY, ROYAL SOCIETY OF CHEMISTRY, UK, vol. 1, no. 11-12, 1 January 2009 (2009-01-01), pages 625-634, XP002663338, ISSN: 1757-9694, DOI: 10.1039/B918172P [retrieved on 2009-10-14]
- DU, Z ET AL.: 'Microfluidic-based Diagnostics for Cervical Cancer Cells.' BIOSENSORS AND BIOELECTRONICS. vol. 21, 19 October 2005, pages 1991 - 1995, XP024961676
- ALBRECHT, D ET AL.: 'Photo- and Electropatterning of Hydrogel-encapsulated Living Cell Arrays.' LAB ON A CHIP. vol. 5, 24 November 2004, pages 111 - 118, XP055182383
- LUTOLF M P ET AL: "Perturbation of single hematopoietic stem cell fates in artificial niches", INTEGRATIVE BIOLOGY,, vol. 1, no. 1, 1 January 2009 (2009-01-01) , pages 59-69, XP002608475, ISSN: 1757-9694, DOI: 10.1039/B815718A [retrieved on 2008-11-21]

## Description

### BACKGROUND OF THE DISCLOSURE

Over the last decade there have been great gains in the fields of genetics, cellular and molecular biology. However, despite this increase in knowledge in the biological sciences there has not been corresponding advances in the ability to diagnosis or prognosis a disease, or predict a patient's response to a line of therapy.

There is growing evidence that three-dimensional organization of a cell dictates cell behavior and function *in vitro.* Thus, analyzing a cell's cellular response in adherent, two-dimensional cultures may not be appropriate for determining a cell's response to drug treatment.

The present disclosure provides systems, devices, and methods for determining a disease state, predicting response to therapeutic treatment, prognosis, diagnosis, and clinical management of a subject suffering from a disease from a complex biological sample at a resolution level of a single cell in a three-dimensional matrix.
US 2012/0149781 discloses stem cell differentiation using light-responsive hydrogels.
US 2010/0028928 discloses a device for high-throughput stimulation, immunostaining and visualization of single cells.

### SUMMARY OF THE DISCLOSURE

The present disclosure provides a method for detecting a response of a live cell comprising the steps of contacting a live cell to a cell capture array device, wherein said cell capture array device comprises a cell microenvironment and an inducible agent; capturing said live cell in said cell microenvironment; inducing the release of said inducible agent into said cell microenvironment; and detecting a response of said live cell. In some embodiments the method for detecting a response of a live cell, comprises a cell microenvironment that further comprises a cell capture moiety. In some embodiments the method for detecting a response of a live cell comprises a cell microenvironment that comprises a hydrogel composition. In some embodiments the method for detecting a response of a live cell, comprises a hydrogel cell microenvironment wherein said live cell is suspended in said hydrogel cell microenvironment. In some embodiments the method for detecting a response of a live cell comprises a cell hydrogel microenvironment that is crosslinked. In some embodiments the method for detecting a response of a live cell comprises a cell hydrogel microenvironment further comprises nutrients suitable to maintain cell viability. In some embodiments the method for detecting a response of a live cell comprises a cell microenvironment that is suitable for maintaining viability of said live cell. In some embodiments the method for detecting a response of a live cell comprises a cell microenvironment that is of a size suitable to accommodate an individual live cell. In some embodiments the method for detecting a response of a live cell comprises a cell microenvironment that is of a size suitable to bind a target cell based on the size of said target cell. In some embodiments the method for detecting a response of a live cell comprises a cell microenvironment that is at least 10 µm in diameter. In some embodiments the method for detecting a response of a live cell comprises a cell microenvironment that is less than 100 µm in diameter. In some embodiments the method for detecting a response of a live cell comprises a cell microenvironment and cell capture moiety, wherein said cell capture moiety is a peptide, a protein, a small molecule, a ligand, an antibody, a receptor, a nucleic acid, a glycoprotein, an oligosaccharide, and combinations thereof. In some embodiments the method for detecting a response of a live cell comprises a cell microenvironment and further comprises a cell capture moiety, wherein said cell capture moiety is an antibody. In some embodiments the method for detecting a response of a live cell comprises a cell microenvironment further comprises a cell capture moiety, wherein said cell capture moiety is an antibody that recognizes a cancer cell. In some embodiments method for detecting a response of a live cell comprises an inducible agent that is of a known concentration. In some embodiments the method for detecting a response of a live cell comprises an inducible agent that is a cancer therapeutic. In some embodiments the method for detecting a response of a live cell comprises an inducible agent that is a prospective cancer therapeutic. In some embodiments the method for detecting a response of a live cell further comprises recording said response of said live cell using a processor instructed by a computer-readable medium. In some embodiments the method for detecting a response of a live cell comprises a live cell that is obtained from a subject. In some embodiments the method for detecting a response of a live cell comprises an inducible agent that further comprising comparing said detected response of said live cell to a reference.

The present disclosure provides a method for capturing a live target cell comprising: contacting a biological sample to a cell capture array device, wherein said cell capture array device comprises a cell microenvironment, an inducible agent, and a cell capture moiety; and capturing a target cell with said cell capture moiety from said biological sample, wherein said target cell is suspended in said cell microenvironment. In some embodiments the method for capturing a live target cell comprises a cell microenvironment that is suitable for maintaining viability of said target cell. In some embodiments the method for capturing a live target cell comprises a cell microenvironment that comprises a hydrogel. In some embodiments the method for capturing a live target cell comprises further comprising crosslinking said hydrogel, wherein said crosslinking forms a 3-D cell microenvironment. In some embodiments the method for capturing a live target cell further comprises a cell capture, wherein said cell capture moieties are different from one another. In some embodiments the method for capturing a live target cell further comprises a cell capture moiety, wherein said cell capture moiety is a peptide, a protein, a small molecule, a ligand, an antibody, a receptor, a nucleic acid, a glycoprotein, an oligosaccharide, and combinations thereof. In some embodiments the method for capturing a live target cell comprises a cell capture moiety that is an antibody. In some embodiments the method for capturing a live target cell comprises a cell capture moiety that is an antibody that recognizes a cancer cell. In some embodiments the method for capturing a live target cell further comprises inducing the release of said inducible agent into said cell microenvironment. In some embodiments the method for capturing a live target cell comprises an inducible agent that is a cancer therapeutic. In some embodiments the method for capturing a live target cell comprises an inducible agent is a prospective cancer therapeutic. In some embodiments the method for capturing a live target cell comprises a biological sample that is obtained from a subject.

The present disclosure also provides for various devices. Specifically, the disclosure provides for a cell capture array device comprising: a lid gasket comprising an inlet and an outlet connect to a microchannel; wherein said lid gasket is connected to a bottom substrate; and a bottom substrate comprising a cell microenvironment, wherein said cell microenvironment comprises an inducible agent. In some embodiments the cell capture array device has a cell microenvironment that further comprises a cell binding moiety. In some embodiments the cell capture array device has a cell microenvironment that is suitable for binding an individual cell. In some embodiments the cell capture array device has a cell microenvironment that is suitable to maintain cell viability. In some embodiments the cell capture array device has a cell microenvironment that is of a size suitable to accommodate an individual cell. In some embodiments the cell capture array device has a cell microenvironment that is at least 10 µm in diameter. In some embodiments the cell capture array device has a cell microenvironment that is less than 100 µm in diameter. In some embodiments the cell capture array device has a cell microenvironment further comprising a cell binding moiety wherein in said capture moiety is a peptide, a protein, a small molecule, a ligand, an antibody, a receptor, a nucleic acid, a glycoprotein, an oligosaccharide, and combinations thereof. In some embodiments the cell capture array device has a cell microenvironment further comprising a cell binding moiety wherein said capture moiety is an antibody that recognizes a cancer cell. In some embodiments the cell capture array device has a cell microenvironment wherein said inducible agent is a cancer therapeutic. In some embodiments the cell capture array device has a cell microenvironment wherein said inducible agent is a prospective cancer therapeutic. In some embodiments the cell capture array device has a cell microenvironment configured in an array format suitable for contact with an agent transfer device. In some embodiments the cell capture array device has a cell microenvironment wherein said inducible agent is array further comprises a solid substrate. In some embodiments the cell capture array device has a cell microenvironment and is further comprised of a solid substrate, wherein said solid substrate comprises silica, silicon, quartz, or combinations thereof. In some embodiments the cell capture array device is further comprises a surface surrounding said microenvironment, wherein said surface surrounding said microenvironment comprises a material that prohibits cell binding. In some embodiments the cell capture array device is further comprises a cell microenvironment that is on an insertable slip.

The present disclosure provides for a cell filtration device comprising: a top chamber comprising an inlet and an outlet; and top chamber connected to a bottom surface comprising a plurality of obstacles thereby providing filtration. In some embodiments the cell filtration device further comprises a bottom surface that has a cell microenvironment. In some embodiments the cell filtration device comprises a plurality of obstacles that is in a form a channel, a island, a post, or combinations thereof.

The present disclosure provides a method of agent transfer to a cell comprising: dispensing an agent onto an device comprising a microenvironment capable receiving said agent to generate an agent transfer device; and contacting said microenvironment of said agent transfer device to a cell microenvironment of a cell capture array, wherein said cell capture array comprises captured cells, and wherein said contacting allows said agent to be transferred to said captured cell. In some embodiments the method of agent transfer to a cell comprises an agent which is a cancer therapeutic.
In some embodiments the method of agent transfer to a cell comprises an agent which is a prospective cancer therapeutic. In some embodiments the method of agent transfer to a cell comprises a captured cell which is a live cell.

The present disclosure provides a method of determining a subject's disease state comprising: contacting a live cell to an cell capture array device, wherein said cell capture array device comprises a cell microenvironment and an inducible agent; capturing said live cell in said cell microenvironment; inducing the release of said inducible agent; detecting a response of said live cell; and comparing said live cell response to a profile derived from a reference cell, and determining the disease state of said subject.

The present disclosure provides for a kit comprising: a cell capture array device or an agent transfer device; and written instructions. In some embodiments the kit contains written instructions that explain the use of the kit for determining a therapeutic response of a subject. In some embodiments the kit contains written instructions that explain use of the kit for determining the disease state of a subject. In some embodiments the kit contains written instructions that explain the use of the kit for characterizing the cellular response of a cell.

A cell capture array for characterizing a cell comprising, a solid substrate, an array of cell-philic sites that is substantially compatible with binding of a single live cell, wherein said cell-philic site is further comprised of an immobilized activatiable-release stimulus.

A cell capture array for characterizing a cell comprising a solid substrate, an array of cell-philic sites that is substantially compatible with binding of a single live cell, wherein said cell-philic site further comprises an immobilized activatiable-drug release stimulus that allows for assaying a single live cell signaling response in said cell.

A cell capture array for characterizing a cell comprising, a solid substrate; an array of cell-philic sites that is substantially compatible with binding of a single live cell, wherein said cell-philic site further comprises an activatiable-release stimulus of known concentration to interact with a single live cell.

A cell capture array for characterizing a cell comprising, a solid substrate; an array of cell-philic sites that is substantially compatible with binding of a cell, wherein said cell-philic site further comprises an activatiable-release stimulus that allows for phenotyping and enumeration of said cell. In some embodiments, said cell is a eukaryotic cell. In some embodiments said cell is a prokaryotic cell. In some embodiments said cell-philic site further comprises peptide, protein, small molecule, cell surface ligand, antibody, receptor or combinations thereof. In some embodiments the surface surrounding said cell-philic site is substantially prohibitive to cell binding. In some embodiments said cell-philic site is at least 10 µm in size. In some embodiments said cell-philic site is no larger than 100 µm in size. In some embodiments said cell-philic site further comprises nutrients suitable to maintain cell growth. In some embodiments said cell-philic site are in an arrayed format on said solid substrate. In some embodiments there is more than one said activatiable-release stimuli. In some embodiments said solid substrate comprises a silica-containing material. In some embodiments said activatiable-release stimulus is of one or varying known concentrations. In some embodiments said activatiable-release stimulus is attached to the said cell-philic site, to a bead, or a combination thereof. In some embodiments, the array is further comprised of microfluidic channel that allows for contacting said individual live cell with reagents for analyzing said individual live cell. In some embodiments said reagents for analyzing said individual live cell are labeled for optical, electrical or magnetic detection. In some embodiments wherein said cell is characterized for a response to said controlled release stimulus on said array. In some embodiments said cell is released from said cell-philic site and characterized for a response not on said array. In some embodiments said stimulus comprises siRNA, DNA, small molecule inhibitor, small molecule activator, antigen, antibody, peptide, protein or any combination thereof. In some embodiments said cell is an adherent cell, a suspension cell or a combination thereof. In some embodiments said cell is a circulating tumor cell. In some embodiments the array is insertable into a tumor.

A method of determining the response by a subject to a therapeutic agent comprising binding a individual live cell from a subject to the array, wherein said cell-philic site is functionalized with a therapeutic agent of one or varying known concentrations; releasing said therapeutic agent to allow said therapeutic agent to interact with said individual live cell; measuring the response of a signaling pathway modulated by said therapeutic agent in said individual live cell; comparing said signaling pathway response of said individual live cell to a reference; and determining the response by a subject to said therapeutic agent. In some embodiments said cell from a subject is obtained from a biological sample, wherein said biological sample comprises whole blood, blood components, lymph fluid, or combinations thereof. In some embodiments said biological sample is enriched for a cell of interest. In some embodiments said therapeutic agent comprises an anti-cancer drug, or combinations treatments with said anti-cancer drug. In some embodiments said signaling pathway response is cell death, proliferation, growth, cell cycle pathways or combinations thereof. In some embodiments said determining the response by a subject to said therapeutic agent further comprises using clinical information.

A method for screening a therapeutic agent for disease response comprising binding a diseased cell to the array, wherein the array is functionalized with a therapeutic agent of one or varying known concentrations; releasing said therapeutic agent to allow said therapeutic agent to interact with said diseased cell; and measuring signaling pathway response to said therapeutic agent. In some embodiments said therapeutic agent comprises an anti-cancer drug or combination treatment with said anti-cancer drug. In some embodiments said array is surgically inserted in a tumor of a subject and monitored for response.

A method for determining a subject's disease state comprising binding an individual live cell from a subject to the array, wherein the array is functionalized with a therapeutic agent of one or varying known concentrations; releasing therapeutic agent to allow said therapeutic agent to interact with said individual live cell; measuring said individual live cell's signaling pathway response; comparing to said individual live cell's signaling pathway response to a signaling profile derived from a reference cell; and determining the disease state of said subject. In some embodiments In some embodiments said individual live cell from a subject is obtained from a biological sample, wherein said biological sample comprises whole blood, components of blood, lymph fluid or combinations thereof. In some embodiments said biological sample is enriched for the cell of interest. In some embodiments said live cell is a circulating tumor cell. In some embodiments said disease state is proliferative cell disease. In some embodiments said disease state is cancer. In some embodiments said disease state is metastatic cancer. In some embodiments said determining disease state of a subject further comprises using clinical information.

A method of analyzing a live cell from a tumor biopsy comprising obtaining a tumor biopsy from a subject; contacting said tumor biopsy to an array of the present disclosure; releasing said controlled release stimulus to interact with said tumor biopsy; and measuring signaling pathway response of said tumor biopsy. In some embodiments said controlled release stimulus is an anti-cancer drug or combination treatment with said anti-cancer drug. In some embodiments said tumor biopsy is derived from a proliferative cell disease. In some embodiments said tumor biopsy is derived from cancer. In some embodiments said tumor biopsy is derived from metastatic cancer.

A kit comprising an array; a gasket with inlet and outlet ports; ligand insert slips; and written instructions on uses of said array. In some embodiments said written instructions explain the use of the kit for determining a therapeutic response of a subject. In some embodiments wherein said written instructions explain the use of the kit for determining the disease state of a subject. In some embodiments the devices and method provided herein are used for monitoring disease state of a subject. In some embodiments said written instructions explain the use of the kit for characterizing the signaling pathway response of a live circulating tumor cell.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:
**FIG. 1** illustrates embodiments of a cell capture array device. **1A** illustrates a diagram of a cell capture array device comprising arrayed cell-philic sites and cell-phobic surface. **1B** illustrates a cell capture array device with graded hydrophobicity surfaces **101** shows a target cell capture sites (e.g. cell-philic surface) at the edge having greater hydrophobicity **102** target cell capture sites at the center having lower hydrophobicity **103** solid substrate, and **104** cell-phobic surface.
**FIG. 2** illustrates an exemplary embodiment of cell capture array device with a 3-D cell-philic surface or cell microenvironment.
**FIG. 3** illustrates several embodiments of exemplary configurations of surfaces that can be used to create the devices provided herein.
**FIG. 4** illustrates a side view of a cell capture array device comprising a gasket with inlet and outlets that allows for flow of a biological sample across the surfaces of the device, capture of target cells, and expelling of non-target cells.
**FIG. 5** illustrates an embodiment of a cell capture array device with different cell-philic surfaces comprising different types of cell binding moieties, different types of thcrapcutics, and/or different concentrations of a thcrapcutics.
**FIG. 6** illustrates an embodiment of a cell-philic surface of a cell capture array device. **6A** depicts a 2-D cell-philic surface with an activatiable-release stimuli and a cell binding moiety (Cl). **6B** depicts a 3-D cell-philic surface with an activatiable-release stimuli and a cell binding moiety.
**FIG. 7** illustrates two embodiments of cell analysis using a tumor biopsy device. **7A** illustrates an embodiment of a method of measuring cell response from a needle biopsy sample. **7B** illustrates an embodiment of a method of measuring cell response a tissue biopsy.
**FIG. 8** illustrates an embodiment of a high-throughput drug screening method using cell capture array device and an agent transfer device. **8A** illustrates an embodiment of a cell capture array. **8B** illustrates an embodiment of an agent transfer array. **8C** illustrates an embodiment of a method of drug delivery and screening by contacting the surfaces of a cell capture array device with the surfaces of an agent transfer array.
**FIG. 9** illustrates various embodiments of a cell filtration device for separating cells of different sizes or different features. **9A** illustrates a cell filtration device with an overlay of channels. **9B** illustrates a cell filtration device with islands of various sizes. **9C** illustrates a cell filtration device with an overlay of channels and graded islands of various sizes.
**FIG. 10** illustrates various embodiments of a cell filtration device. **10A** illustrates a filtration device with **1010** target cell capture sites, **1020** cell filtration channels, and **1030** surface of hydrophilic region. **10B** illustrates cell filtration device with **1040** target cell capture sites and **1050** cell filtration islands.
**FIG. 11** illustrates an embodiment of a polymer that can be used with the surfaces of the devices provided herein.
**FIG. 12** illustrates two embodiments of methods of cell analysis using the cell capture array device. **12A** depicts the cell analysis method of capture and release (*e.g*., off-array). **12B** depicts the cell analysis method of capture and measuring a cell's response on the device (*e.g.,* on-array).
**FIG. 13** illustrates an embodiment of a device provided by the disclosure integrated with a system comprising a processor instructed by computer-readable medium means.
**FIG. 14** illustrates an embodiment of a method of manufacture to create the devices provided herein.
**FIG. 15** illustrates an embodiment of a method of manufacture to produce a cell capture array or agent transfer device.
**FIG. 16** illustrates results investigating cell viability with the cell capture array device.
**FIG. 17** illustrates dose-dependent drug treatment study with single live cells on a cell capture array device. **17A** shows a cell capture array device with the majority of the cell-philic sites being occupied by an individual live cell. **17B** shows an agent transfer device loaded with increasing concentrations of ligand. **17C** shows results from a dose-dependent response study using a cell capture array device.
**FIG. 18** illustrates fluid flow behavior on various embodiments of a cell filtration device. **18A** shows fluid flow behavior as a function of flow between a top and a bottom plate. **18B** shows back-pressure as a function of flow separation into predefined channels. **18C** shows flow separation into channels as a function of constant spacing. **18D** shows back-pressure as a function of flow spillage out of predefined channels. **18E** shows flow separation into channels as a function of channel spacing.
**FIG. 19** illustrates particle separation on various embodiments of a cell filtration device. **19A** shows particle separation by size as a function of spacing between posts or islands. **19B** shows particle separation by size as a function of difference in particle size for a bimodal distribution.

### DETAILED DESCRIPTION OF THE DISCLOSURE

### I. DEFINITIONS

As used herein, the singular forms "a," "an," and "the" are intended to include the plural forms as well, unless the context clearly indicates otherwise. Furthermore, to the extent that the terms "including," "includes," "having," "has," "with," "such as," or variants thereof, are used in either the specification and/or the claims, such terms are not limiting and are intended to be inclusive in a manner similar to the term "comprising".

The term "array" can refer to a plurality of spatially arranged domains. The plurality of spatially arranged domains can be set forth in a number of configurations.

The terms "assay," "assaying" or like terms generally refer to an analysis or test. An assay can be used to determine, for example, the presence, absence, quantity, extent, kinetics, dynamics, or type of a cell's response upon stimulation with a stimuli, such as a ligand candidate

The terms "attach," "adhere," "immobilized", or like terms generally refer to a method of fixing a position or restricting the freedom of movement of an object, such as a molecule or cell. Restricting movement can be accomplished by any method known in the art, some non-limiting examples can include: physical separation, physical absorption, chemical bonding (*e.g*. covalent bonds, ionic bonds, the London dispersion force, or hydrogen bonding), electrostatic force, or electromagnetic force or any combination thereof. For example, a surface modifier substance, a cell, a ligand candidate compound, and like entities of the disclosure, can attach to a surface, through forces such as such as by physical absorption, chemical bonding, and like processes, or combinations thereof.

The term "cell attachment," "cell adhesion," or like terms refer to the interacting or binding of cells to a surface. Cell attachment can be accomplished, for example, by any known method in the art including: cell culturing, interaction with cell anchoring materials (e.g., fibronectin, collagen, lamin, gelatin, polylysine, etc.), by a ligand-cell surface interaction, or any combination thereof.

The term "adherent cells" refers to a cell or a cell line or a cell system, such as a prokaryotic or eukaryotic cell, that remains associated with, immobilized on, or in certain contact with the outer surface of a substrate. "Suspension cells" refers to a cell or a cell line that is preferably cultured in a medium wherein the cells do not attach or adhere to the surface of a substrate during the culture. However some cells can have both adherent and suspension properties, some circulating tumor cells can exhibit both adherent and suspension properties. In some applications, the presence of both adherent and suspension properties can correspond to a cell undergoing a transition between epithelial and mesenchymal states.

The term "cell culture" or "cell culturing" refers to the process by which either prokaryotic or eukaryotic cells are grown under controlled conditions. "Cell culture" not only refers to the culturing of cells derived from multicellular eukaryotes, especially animal cells, but also the culturing of complex tissues and organs.

The term "cell" or like term refers to a small usually microscopic mass of protoplasm bounded externally by a semipermeable membrane, optionally including one or more nuclei and various other organelles, capable alone or interacting with other like masses of performing all the fundamental functions of life, and forming the smallest structural unit of living matter capable of functioning independently including synthetic cell constructs, cell model systems, and like artificial cellular systems.

The term "cell system" or like term refers to a collection of more than one type of cells (or differentiated forms of a single type of cell), which interact with each other to performing a biological or physiological or pathophysiological function. For example, a cell system can include but is not limited to more than one cells types in a culture system, an whole or portion of an intact organ, a tissue slice or a portion from a tissue, a circulating tumor cell in blood or the like.

The term "single cell" refers to one cell. Single cells can be useful in the methods described herein can be obtained from a cell culture (*e.g*. bacterial, tissue, mammalian), a body fluid, a tissue of interest, a biopsy, a blood sample, a lymph sample, or the like. Furthermore, in general, cells from any population can be used in the methods, such as a population of prokaryotic or eukaryotic single celled organisms including bacteria or yeast,

The term "detect" or like terms can refer to an ability of the device and methods of the disclosure to discover or sense a cellular response. In some applications, the cellular response is the response from a stimulus, furthermore, in some applications detection can include the ability to distinguish the sensed responses for distinct stimuli.

The term "functionalize", "functionalizing or like terms refer to modification of a surface in order to achieve desired properties such cell attraction or cell repulsion. In addition, functional groups can be used to link functional molecules, such as, antigens, antibodies, beads, chemicals to further specialize a surface or a device to a particular purpose. Preferably, a functionalized surface material, can chemically bind a cell-binding agent, such as an antibody or polynucleotide, that is selected to selectively bind a target in a biological sample such as a living cell, organelle, ect.

The term "stimulus," or like terms refers to a biological, pharmaceutical, or chemical compound, agent, or other moiety. Non-limiting examples include simple or complex organic or inorganic molecule, a peptide, a protein, an oligonucleotide, an antibody, an antibody derivative, antibody fragment, a vitamin derivative, a carbohydrate, a toxin, or a chemotherapeutic compound. Various compounds can be synthesized, for example, small molecules and oligomers (*e.g*., oligopeptides and oligonucleotides), and synthetic organic compounds based on various core structures. In addition, various natural sources can provide compounds for screening, such as plant or animal extracts, and the like. A skilled artisan can readily recognize that there is no limit as to the structural nature of the agents of the present disclosure.

The terms "subject," "individual," or "patient" are used interchangeably herein, and refer to a vertebrate, for example a mammal, including a human. Mammals include, but are not limited to, murines, simians, humans, farm animals, sport animals, and pets. Tissues, cells and their progeny of a biological entity obtained *in vitro* or cultured *in vitro* are also encompassed.

The term "signal pathway" is a process during which stimulatory or inhibitory signals are transmitted into and within a cell to elicit an intracellular response. A modulator of a signal transduction pathway refers to a compound, which modulates the activity of one, or more cellular proteins mapped to the same specific signal transduction pathway. A modulator may augment (agonist) or suppress (antagonist) the activity of a signaling molecule.

The terms "co-administration," "administered in combination with," and their grammatical equivalents, as used herein, encompasses administration of two or more agents to a subject so that both agents and/or their metabolites are present in the subject at the same time. Co-administration includes simultaneous administration in separate compositions, administration at different times in separate compositions, or administration in a composition in which both agents are present.

The term *"in vivo"* refers to an event that takes place in a subject's body.

The term *"in vitro"* refers to an event that takes places outside of a subject's body. For example, an in vitro assay encompasses any assay run outside of a subject assay. *In vitro* assays encompass cell-based assays in which cells alive or dead are employed. *In vitro* assays also encompass a cell-free assay in which no intact cells are employed.

The term "label" refers to a molecule or an aspect of a molecule that can be detected by physical, chemical, electromagnetic and other related analytical techniques. Examples of detectable labels that can be utilized include, but are not limited to, radioisotopes, fluorophores, chromophores, mass labels, electron dense particles, magnetic particles, spin labels, molecules that emit chemiluminescence, electrochemically active molecules, enzymes, cofactors, enzymes linked to nucleic acid probes and enzyme substrates.

The term "labeling reagent" can be a reagent that is capable of binding to an analyte, being internalized or otherwise absorbed, and being detected, e.g., through shape, morphology, color, fluorescence, luminescence, phosphorescence, absorbance, magnetic properties, or radioactive emission.

The terms "cell-philic site" or "cell microenvironment" can be a surface that is capable of cell binding or cell binding and maintaining a cell in a viable state such that it can undergo growth and division.

### II. OVERVIEW

The present disclosure provides systems, methods, and devices for testing and measuring a single live cell or particle's response to a stimulus (*e.g* therapeutic, ligand, antibody, ect.). The devices provided by the present disclosure allow for capture and isolation of a particular cell type of interest, enrichment of a cell population, and a multiplexed profiling assay of a biological sample at the single cell level. The methods provided herein allow for a single live cells or particles to be analyzed on or off the device including but not limited cellular analysis such as morphology, shape, cell signaling responses, migratory ability (*e.g*. EMT) status, proliferative capacity, cell death response, kinetic properties, drug response, and/or other information of potential relevance such as genotyping by RNA or DNA sequencing, transformation studies, therapeutic screen studies such as translational *in vitro* clinical testing, proteomics studies, and cell linage and cell response time-based analysis.

The devices and methods provided herein allow for a disease state of a biological sample to be determined, wherein single cells or particles are assayed for phenotype, genotype, and their cellular response to a stimulus thereby providing information to determine a disease state of an individual. The devices and methods provided herein, allow for clinical management of a patient suffering from a disease or to determine which treatment option might be most effective for an individual suffering from a disease; for example, a response of a primary cancer cell to one or more anti-cancer therapeutics may be tested to determine which therapeutic regimen is most effective in killing the cancer cell. The devices and methods provided also to evaluate and determine a patient's prognosis or diagnosis, those at risk for a disease, and a patient's response to a therapeutic of disease by a characterization from a biological sample a patient suffering from a disease wherein the diseased cell and its responses are observed, measured and/or quantified to characterize the disease or disease state. Furthermore, the devices and methods provided herein allow for the characterization of a disease cell which can be used to determine the presence or absence of cell biomarker markers, genotype status such as a mutation, or response to a stimulus such as a therapeutic agent to generate a diagnostic test or prognostic test for a disease or help direct drug development. In addition, the devices and methods provided herein allow for the *in vitro* drug response screening to help direct clinical management and drug development efforts.

Thus, systems, methods, and devices of the disclosure stand to aid basic researchers, drug developers, and clinicians in directing drug development, prediction of drug response and resistance in a patient and will help direct the best course of clinical management of a patient suffering from evolving and progressive disease, such as cancer.

### III. DEVICES

### A. Single Cell Capture Array

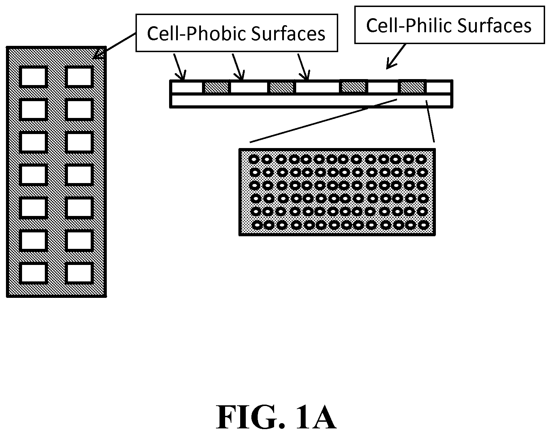

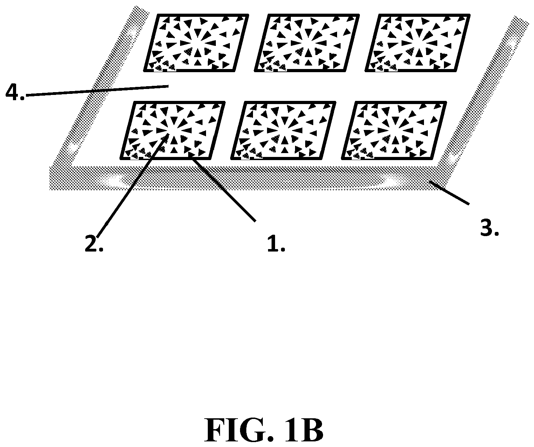

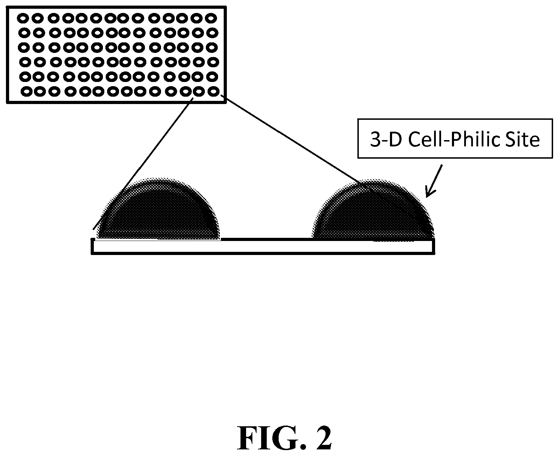

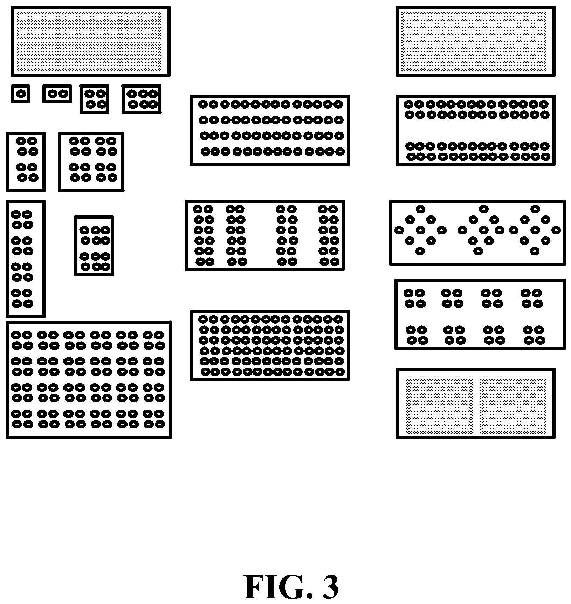

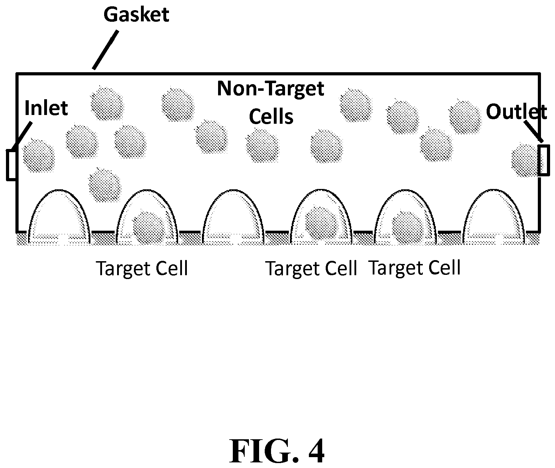

The present disclosure provides various devices used for single cell capture and characterization. In one embodiment, the present disclosure provide a device for live cell capture comprising: a solid substrate that is connected to a hydrogel composition, wherein said hydrogel composition comprises a crosslinkable agent, a moiety capable of specifically binding to a particular cell type, and an inducible agent (*e.g*. drug, therapeutic, antibody, stimuli, ligand, or small molecule inhibitor); an inlet connected to a microchannel gasket wherein said microchannel gasket is capable of receiving a biological sample into said microchannel gasket, wherein said biological sample comprises a live target cell and a non-targeted component; and an outlet connected to said microchannel gasket that allows for expelling of said non-target component of said biological sample. Non-limiting examples of various embodiments of a live cell capture array are illustrated in **FIG. 1****,** **FIG. 2****,** **FIG. 3****,** and **FIG. 4****.** **FIG. 1** illustrates an embodiment of a single live cell capture array device showing the solid support, cell phobic coating and an arrayed configuration of cell-philic sites. **FIG. 2** illustrates an embodiment of a single live cell capture with a protruding two-dimensional or three-dimensional cell-philic surface, where the cell is captured and suspended in said cell-philic surface adjacent to a solid support. **FIG. 3** illustrates various non-limiting embodiments of configurations of cell-philic sights on live cell capture device. **FIG. 4** illustrates a side view of a single live cell capture array device with a gasket with inlet and outlets which can allow for flow of cells across the array device so that the target cells can be captured as they pass the protruding cell-philic surfaces.

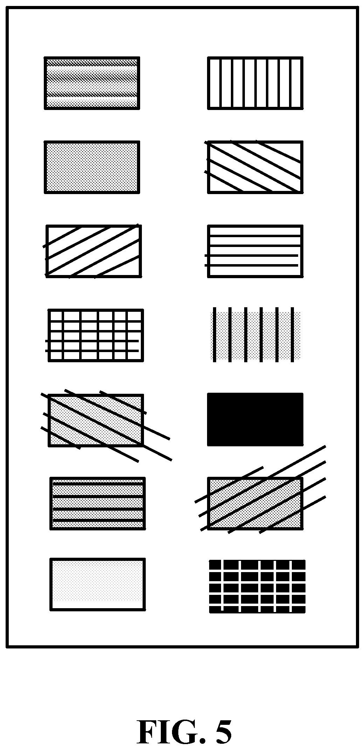

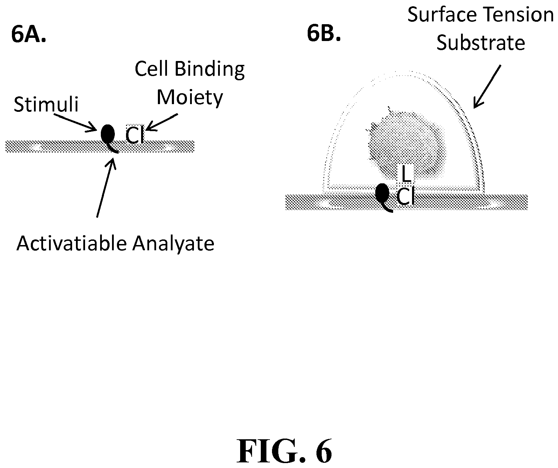

**FIG. 5** illustrates an embodiment of live cell capture array device where different types of cell-philic surfaces may be arrayed on a single cell capture array device. **FIG. 6** illustrates an embodiment of the single cell capture surface: **6A** depicts an inducible-release stimuli (*e.g*. drug, therapeutic, antibody, stimuli, ligand, or small molecule inhibitor) with a cell binding moiety or antigen (Cl) on a cell-philic surface. **6B** depicts a protruding cell-philic surface created by surface tension on a single cell capture array device. The protruding cell-philic surface can be two-dimensional or three-dimensional depending on the application. In some applications, the captured cell from a device can be tracked from its captured place on the device by adding barcoding, staining with a dye, molecular markers, or imaging the captured cell such that it can be tracked after release from the device in downstream cell analysis applications such as RNA or DNA sequencing, transformation, translational studies, proteomics, and cell linage and cell response time-based analysis).

In some applications, the device captures and maintains at least 95%, 90%, 85%, 80%, 75%, 70%, 65%, 60%, 55%, 50%, 45%, 40%, 35%, 30%, 25%, 20%, 15%, 10%, or 5% viable cells. In some applications, the device of captures at least 5%, 10%, 15%, 20%, 25%, 30%, 35%, 40%, 45%, 50%,55% 60%, 65%, 70% , or 75% of target cells in a biological sample. In some applications, the device of captures at least 5%, 10%, 15%, 20%, 25%, 30%, 35%, 40%, 45%, 50%, 55% 60%, 65%, 70% , or 75% of target cells in an enriched sample.

In some applications, the device of excludes at least 5%, 10%, 15%, 20%, 25%, 30%, 35%, 40%, 45%, 50%, 55% 60%, 65%, 70% , or 75% of non-targeted cells in a biological sample. In some applications, the device of excludes at least 5%, 10%, 15%, 20%, 25%, 30%, 35%, 40%, 45%, 50%, 55% 60%, 65%, 70% , or 75% of non-targeted cells in an enriched sample.

### B. In Vivo Device

In some embodiments, the disclosure provides methods and devices for conducting an *in vivo* assay. In this application the device of a size and materials that is capable of being surgically inserted into a subject for use in an *in vivo* assay. In some applications, the device can be inserted into a tumor to determine and/or monitor the response of treatment in real time. In some applications, the device can be inserted into a tumor to determine and/or monitor the response after removal of the device by storage and transfer of the collected treatment response data to a computer.

### C. Tumor Biopsy Device

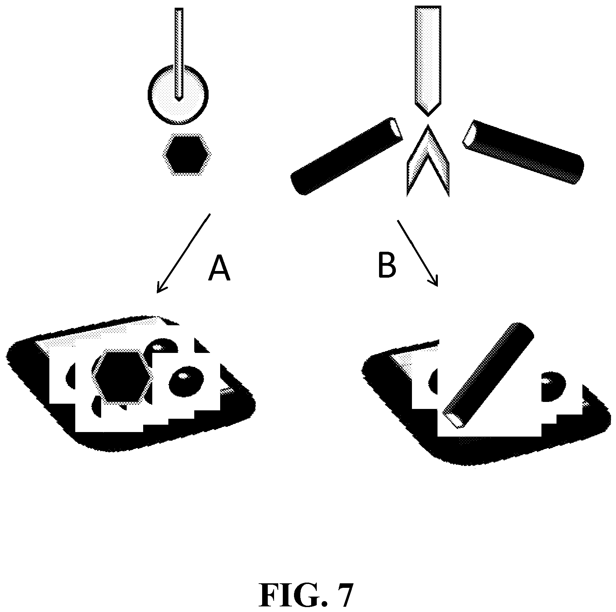

In some embodiments, the disclosure provides methods and devices for conducting *an in vitro* assay on tumor biopsy samples such as tissue obtained from a needle biopsy or whole tissue standard biopsy. Use of a single cell capture array greatly decreases the amount of required material for a diagnostic screen. A small needle biopsy from a tumor with minimal discomfort can used to analyze the cellular content of a tumor for specific biomarkers or cellular response to a therapeutic treatment. It one aspect the disclosure provides methods and devices for single cell capture from a needle biopsy from a solid tumor **(****FIG. 7A****).**

It is contemplated that certain tissue suspected of being diseased, for example a cellular growth suspected of being malignant can be sampled by swabbing or scraping a tissue to obtain a few cells that can be interrogated and analyzed with the methods and devices of the claimed disclosure. It one aspect the disclosure provides methods and devices for single cell capture in a standard tissue biopsy from a solid tumor **(****FIG. 7B****).**

### D. Agent Transfer Device

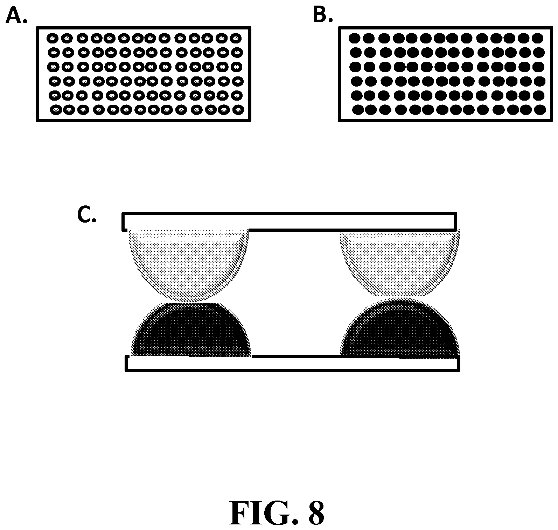

Non-limiting examples of various embodiments of an agent transfer device are illustrated in **FIG. 8B** and **8C****.** In some applications, the present disclosure provides an agent transfer device comprising a solid substrate that is connected to a hydrogel composition, wherein the agent to be transferred is within the hydrogel composition; an inlet connected to a microchannel gasket that allows the receiving and dispending of an agent; and an outlet that allows expelling of any excess agent.

In some applications, the present disclosure provides an agent transfer device comprising a solid substrate and an agent connected to the solid substrate; an inlet connected to a microchannel gasket that allows the receiving and dispending an agent; and an outlet that allows expelling of any excess agent.

In some applications, the agent is transferred by diffusion through a hydrogel composition into another hydrogel composition. In other applications, the agent induced to release from a solid substrate or hydrogel composition thereby allowing the agent to contact a cell.

In some applications, the device of transfers at least 100%, 95%, 90%, 85%, 80%, 75%, 70%, 65%, 60%, 55%, 50%, 45%, 40%, 35%, 30%, 25%, 20%, 15%, 10%, or 5% original concentration of the agent on the device. In some applications, the device of transfers at least 100%, 95%, 90%, 85%, 80%, 75%, 70%, 65%, 60%, 55%, 50%, 45%, 40%, 35%, 30%, 25%, 20%, 15%, 10%, or 5% original concentration of the agent on the device.

### E. Cell Filtration Device

The disclosure provides a cell filtration device that is used to separate cells in a heterogeneous cell population. In some embodiments of the methods provided herein, cell filtration device that is used to separate or enrich cells from a complex, heterogeneous biological sample.

In some embodiments, the cell filtration device uses to cell features (*e.g*., cell surface markers, ect) to separate or enrich cells from a complex heterogeneous biological sample. In some embodiments, cell filtration device uses cell size to separate or enrich cells from a complex, heterogeneous biological sample.

In some embodiments, the cell filtration device comprises a solid substrate connected to a hydrogel composition, wherein said hydrogel composition is in a graded and sieve-like pattern, thereby providing exclusion of different sized cells; an inlet connected to a microchannel gasket allowing dispensing of a biological sample, wherein said biological sample comprises different sized cells; and at least two outlets that allows expelling of said different sized cells.

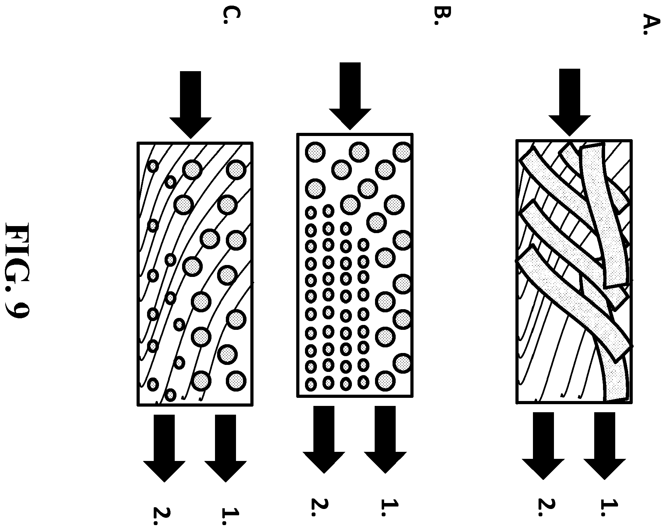

Non-limiting examples of various embodiments of a cell filtration device for separating cells of different sizes are illustrated in **FIG. 9. FIG. 9A** illustrates an embodiment of a cell filtration device with an overlay of channels. **FIG. 9B** illustrates an embodiment cell filtration device with islands of various sizes. **FIG. 9C** illustrates an embodiment cell filtration device with an overlay of channels and graded islands of various sizes.

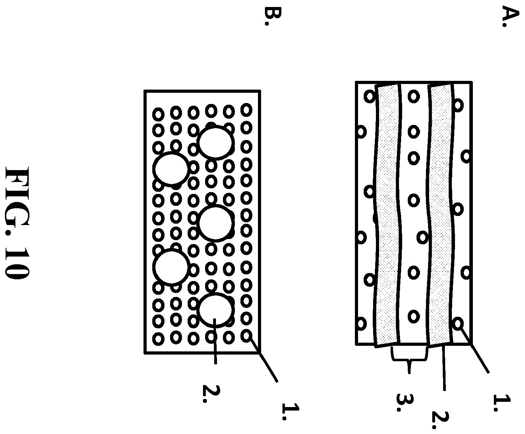

In other embodiments of a cell filtration device can be further comprised of target cell capture sites, cell filtration channels, and surface of hydrophilic region **(****FIG. 10A****).** In other embodiments a cell filtration device can be further comprises target cell capture sites and cell filtration islands **(****FIG. 10B****).**

In some applications, the device filters out at least 100%, 95%, 90%, 85%, 80%, 75%, 70%, 65%, 60%, 55%, 50%, 45%, 40%, 35%, 30%, 25%, 20%, 15%, 10%, or 5% of non-targeted cells. In some applications, the device filters out at least 100%, 95%, 90%, 85%, 80%, 75%, 70%, 65%, 60%, 55%, 50%, 45%, 40%, 35%, 30%, 25%, 20%, 15%, 10%, or 5% of targeted cells.

In other applications, the device filters out at least at least 100%, 95%, 90%, 85%, 80%, 75%, 70%, 65%, 60%, 55%, 50%, 45%, 40%, 35%, 30%, 25%, 20%, 15%, 10%, or 5% of cellular debris. In other applications, the device filters out at least at most 100%, 95%, 90%, 85%, 80%, 75%, 70%, 65%, 60%, 55%, 50%, 45%, 40%, 35%, 30%, 25%, 20%, 15%, 10%, or 5% of cellular debris.

### F. Dimensions of Devices

One skilled in the art can recognize the device may be of a variety of sizes and scalable depending on the desired application or desired computability with various devices to be used in an analysis system.

The device can be approximately the length and width of a standard microscope slide (1 inch width by 3 inches length).

In other applications, the device can be of a size suitable to insert into a tumor of subject. In some applications, the device can be of a size suitable to place a solid tumor sample on. In other applications, the device can be of a size suitable to assay a needle biopsy sample.

### IV. DEVICE MATERIALS

### A. Solid Support Substrate

In some applications, the device can contain a solid support substrate. In other applications, the device will not contain or need a solid support substrate. In instances where a solid support is used, the solid support substrate can be comprised of any suitable material, such as silicon, fused silica, silica-containing materials, glass, float glass, borosilicate, borofloat, plastic, polymeric materials, polydimethylsiloxane (PDMS), sol-gel product reactant or combinations thereof. Suitable plastics that can be used for solid support substrate component of the devices substrate include, but are not limited to, polydimethylsiloxane (PDMS), polymethylmethacrylate (PMMA), polycarbonate, polystyrene, polyethylene teraphthalate, polyacrylamide, agarose, or combinations thereof.

The solid substrate can be comprised of one or more polymeric thermoplastic materials such as commodity or engineered polyolefin polymers or copolymers including, but not limited to, polyacrylics (Lucite, polymethylmethacrylate); polycarbonate (Lexan, Calibre, etc.); polyvinyl chloride, polyethylene, polypropylene, polyethylene terephthalate, cycloolefins (cycloolefin copolymer (COC, or TOPAS), or cycloolefin polymer (COP or Zeonor); polystyrene, epoxies or combinations thereof.

The solid substrate can be a thermosetting plastic, such as epoxies (mixture of epoxide resin with polyamine resin), including fiber-reinforced plastic materials. In some embodiments, the solid substrate could be any of these polymeric materials further functionalized with silica.

The solid substrate can be metallic (gold, silver, platinum, copper, aluminum), metal oxides (copper oxide, aluminum oxide, silver oxide, indium tin oxide, etc.) or combinations thereof; inorganic materials including semiconductor materials and magnetic materials or combinations thereof. In some embodiments, the solid substrate can be a combination of silica, polymeric, metallic, or inorganic listed above.

The solid substrate can be made of natural occurring minerals such as non-silicate and silicates. Examples of silicates that can be used with the disclosure include but are not limited to, feldspars, quartz, olivines, pyroxenes, amphiboles, garnets, or micas.

In certain applications, it may be desirable to use a material that is optically transparent for the solid substrate. In other applications, it may be desirable to use a material that is non-optically transparent for the solid substrate. In some applications, it may be desirable to use a material that is coated with another material (such as a primary amine and similar materials that allows for the creation of or increase of surface tension properties) for the solid substrate. In some applications the solid support comprises a microscope slide.

When the application calls for the size of the array to be the size of a standard microscope slide arrays configured for use with conventional microscopy can be further altered with additional features to facilitate their handling. For example, a cell tray bearing an array may be configured with corners that are rounded or with indentations on the edges to allow easy pick-up or manual manipulation by an operator. Vertical tabs for the cell tray can be provided via the machining, molding, or by bonding that facilitate the use of automated grippers in slide handling robots. The cell tray may also be sized to rest on pedestals formed, for example, within a petri-dish-like holder, permitting ready manipulation.

While these systems, methods and devices will be described by reference to an array sized to fit on a standard microscope slide, it is understood that other sizes and shapes of the array's housing may be produced to fit specific industry demands or applications. While a small physical footprint is advantageous for certain purposes, it would be understood in the art that the housing may be formed in any size or shape to fit a particular piece of apparatus, or to provide a sufficiently large matrix for particular analytic purposes.

### B. Cell Capture Surfaces

It is generally an objective of the disclosure to minimize binding by non-target cells **(****FIG. 4****).** It is contemplated that washing steps using various types of buffers may be required in certain applications to remove other unwanted non-target cells, molecules, or cellular debris that are non-specifically bound to the solid support or to the cell-philic surface sites.

The cell capture surface is the surface upon which an individual or multiple cell living target cells can be attached, immobilized, or bound to. The cell capture surface can capture one or more living cells. A cell-philic site can capture an individual or single, living, target cell.

The cell-philic sites can be separated from each other or cultured together in a defined region. The cell-philic sites can be separated from each other by one or more cell-phobic regions. The array can comprise at least 1, 2, 3, 4, 5, 6, 7, 8, 9, 10, 11, 12, 13, 14, 15, 16, 17, 18, 19, 20, 30, 40, 50, 60, different regions. Different regions can be defined by variation is the cell binding entity, drug stimuli, or by the unique combination of both.

A cell capture surface can contain any number of cell-philic sites. Many different numbers of cell-philic sites may be arranged on a single cell capture surface. The number of cell-philic sites that can be on a cell capture surface can include, but is not limited to: 2, 3, 4, 5, 6, 7, 8, 9, 10, 11, 12, 16, 18, 20, 21, 24, 28, 30, 36, 40, 42, 45, 56, 60, 72, 96, 100, 384, 864, 1000 or more cell-philic sites. In some applications, the number of cell-philic sites that can be on a cell capture surface can include more than 10, more than 50, more than 100, more 250 than 500, more than 1,000, more than 2,000, more than 3,000, more than 4,000, more than 5,000, more than 6,000, more than 7,000, more than 8,000, more than 9,000, more than 10,000, more than 20,000, more than 30,000, more than 40,000, more than 50,000, more than 100,000, more than 500,000 or more than 1,000,000 cell-philic sites.

In some applications, the cell-philic sites protrude such that they occupy more vertical space than the cell-phobic surface of the device **(****FIG. 2****).** In some applications the cell-philic protrusions are created primarily by surface tension of a material. In some applications the cell-philic protrusions are created primarily by surface tension and crosslinking of a material. In some applications the cell-philic protrusions are created primarily crosslinking of a material.

In some applications the cell-philic protrusions are 2-D or 3-D cell-philic surface protrusions. In some applications the 2-D or 3-D cell-philic protrusions are can occupy at least 0.1 nm, 0.2 nm, 0.3 nm, 0.4 nm, 0.5 nm, 0.6 nm, 0.7 nm, 0.8 nm, 0.9 nm than the cell-phobic surfaces of the device **(****FIG. 2** and **FIG. 6B****)**.

In some applications the cell-philic surfaces of the device are in form of a well, such that they occupy space lower or below the cell phobic surfaces (e.g., U.S. Provisional Application No. 61/705914).

The cell capture surface can contain one or more cell-philic sites. The plurality of one or more cell-philic sites can be arranged in an array in various array patterns depending on the application. The skilled artesian will recognize that array of cell-philic sites can be set forth in any number of arrangements. Some non-limiting examples of cell-philic array arrangements are shown in **FIG. 3** and in other figures provided herein. The format of the array allows for parallel cellular detection, analysis, and characterization of a target cell. The array format can allow for the use of slips or frames that form a surface. The array format can allow for full or semi-automation.

In some applications the cell-philic sites are arrayed in an evenly spaced manner. In other instances, the cell-philic sites are not evenly spaced. The cell-philic sites can be arranged in an ordered fashion. The cell-philic sites can be arranged in a horizontal, longitudinal, square, or diagonal format **(****FIG. 3****).** In other instances, the cell-philic sites can be in a random or unordered format.

In some applications the cell-philic surfaces on the device can be grouped. In some applications there can be one or more discreet domain surfaces, such as for example a square or rectangular domain. Each domain surface can comprise one or more cell-philic sites. In some applications, each domain surface can comprise cell-philic sites that are the same (*e.g.,* identical cell capture moiety and stimuli). In other instances, each domain surface can comprise cell-philic sites that are different (*e.g*., different types of cell capture moiety and stimuli). In some applications, each domain surface can comprise cell-phobic regions that are the same. In other instances, each domain surface can comprise cell-phobic regions that are different.

The spacing of the cell-philic sites can be arranged to allow for subsequent detection. In some applications, the spacing between the cell-philic sites can be of about the same diameter as the cell-philic surfaces is contemplated.

In some applications the cell-philic surfaces on the device can be different (*e.g*. size, shape, and composition). Each cell-philic site can be functionalized to immobilize or adhere to one or more specific cell types. In some applications a single cell's interaction with a cell-philic site will cause the single cell to adhere or immobilize to the site. In some applications, a single cell's interaction with both the cell-philic site and the surrounding cell-phobic region will cause the single cell to adhere or immobilize to the cell-philic site. In some applications, cells can be selectively and reversibly bound to the cell-philic sites. Reversible immobilization can allow for release of the cell for collection. Reversible immobilization can also allow for the device to be reusable.

In some applications the cell-philic surfaces on the device can have different sizes. The size of the cell-philic sites will depend on the size of the type of single cell intended to be captured. In some applications, the cell-philic sites on the array are all the same size. In other instances, the cell-philic sites can be a mixture of one or more sizes. In some applications the cell-philic surfaces on the device can have at least 1, 2, 3, 4, 5, 6, 7, 8, 9, 10, 11, 12, 13, 14, 15, 16, 17, 18, 19, 20, 30, 40, 50, or 60 different sizes.

In a device comprising a plurality of cell-philic surfaces, the cell-philic surfaces will preferably have substantially identical dimensions. In applications where a range of different cell types are to be caught by a device, then the cell-philic surfaces will have varying dimensions.

Suitable, non-limiting examples of cell-philic site dimensions include at least 1-5 µm in size, at least 5-10 µm in size, at least 10-15 µm in size, at least 15-20 µm in size, at least 20-25 µm in size, at least 25-30 µm in size, at least 30-35 µm in size, at least 35-40 µm in size, at least 40-45 µm in size, at least 45-50 µm in size, at least 50-55 µm in size, at least 55-60 µm in size, at least 60-65 µm in size, at least 65-70 µm in size, at least 70-75 µm in size, at least 75-80 µm in size, at least 80-85 µm in size, at least 85-90 µm in size, at least 90-95 µm in size, at least 95-100 µm in size, or at least 100-110 µm in size.

Suitable, non-limiting examples of cell-philic site dimensions include at least 1-5 µm in, at least 5-10 µm in diameter, at least 10-15 µm in diameter, at least 15-20 µm in diameter, at least 20-25 µm in diameter, at least 25-30 µm in diameter, at least 30-35 µm in diameter, at least 35-40 µm in diameter, at least 40-45 µm in diameter, at least 45-50 µm in diameter, at least 50-55 µm in diameter, at least 55-60 µm in diameter, at least 60-65 µm in diameter, at least 65-70 µm in diameter, at least 70-75 µm in diameter, at least 75-80 µm in diameter, at least 80-85 µm in diameter, at least 85-90 µm in diameter, at least 90-95 µm in diameter, at least 95-100 µm in diameter, or at least 100-110 µm in diameter.

In some applications, cell-philic sites can be designed to mimic the diameter of a particular cell type or particle (*e.g.* cell, organelle or microorganism) size.

### C. Cell-Philic Surfaces

Hydrogels are hydrated, porous materials, which have found utility in cell culture methods. Three-dimensional hydrogels are particularly useful in cell applications, for example for growing or maintain cells in a viable state to analyze their behavior in terms of growth and differentiation and response to external factors such as antigens or therapeutic drugs.

The present disclosure provides for cell-philic surfaces to be made from a hydrogel composition. Suitable hydrogel composition to be used with the device should not be cytotoxic, can be biocompatible (*i.e.* will provide sufficient nutrient and support for the cell to maintain viability and conduct its normal cell response), and can be capable of being degraded or remove such that a cell is release a cell from without harming its structure or state.

The methods used to release a cell from a cell-specific binding agent (*e.g*., binding entity) can have different characteristics from the solution used for binding of said binding agent. That is, the release solution may have a different pH, a different ionic strength, a different temperature and/or it may comprise organic solvents (preferably only at non-toxic concentrations), detergents, chaotropes, or other denaturing reagents, and combinations thereof than the binding solution. For example, the different characteristics of the release solution can causes a change in conditions which results in the affinity of the ligand for the binding agent being greatly reduced, thereby releasing the binding agent and cellular target bound from the binding surface or cell-specific binding agent.

There are several materials can be used to make hydrogels, including but not limited to peptide amphiphiles, fibrillizing peptides, systems that oligomerize through alpha-helical coiled coils, self-assembling proteins, collagen-mimetic systems, polymer-peptide conjugates. Frequently the hydrogel will comprise of a crosslinking agent. Examples of suitable crosslinking agents that can be used include but are not limited to, Bis Acryl PEG 10K (Acryl - PEG 10K - Acryl) and Acryl/NHS PEG3,4K (Acry - PEG 3.4 K - NHS), and Bis acryl PEG10K polymer. By controlling the ratios of the polymers that comprise the hydrogel, such as controlling the ratio between the acryl with the mono acryl polymers, the amount of crosslinking hydrogel and in turn the viscosity of the hydrogel can be tailored to the requirements of a particular cell type, particle, microorganism, or application.

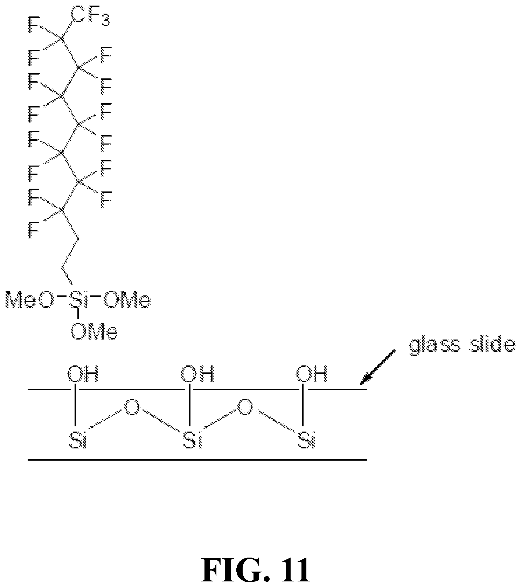

In some applications, the cell-philic surfaces can be formed by covalently adhering polyethylene glycol (PEG) to a surface by first functionalizing the surface with an acrylate moiety, and then covalently binding a diacrylate PEG derivative to the functionalized surface **(****FIG. 11****).** The polyethylene glycol (PEG) material bound to the surface can then be contacted with a cell-binding agent under conditions effective to bind the cell-binding agent to the polyethylene glycol (PEG) material, thereby forming the cell capture surface. In some applications, the cell-philic surfaces are additional functionalized with one or more inducible-release stimulus of known concentration.

Cell-philic surfaces can be made from gel-coated surfaces. Gel-coated surfaces can be further enhanced with nutrients capable of maintaining a living cell's growth for several day, week or months. Suitable gel-coated surfaces that can be used with the claimed disclosure include, but are not limited to polyacrylamide, agarose, polysaccharide polymer material and those provided in U.S. Application No. 61/705914.

In certain instance is may be advantage to change the cell-philic to various sizes, patterns, or scaffolds types. In some applications the cell-philic surfaces are made to form 3-D scaffolds, and other applications the cell-philic surfaces are made to form 2-D scaffolds. In some applications the cell-philic surfaces may be of any suitable size or shape. For example, cell-philic surfaces can be round in shape, triangular, square, rectangular, cylindrical, square or polygonal and a combination of shapes. In order to control the size and shape of the cell-philic surfaces the successive additions of the desired polymer can be used to tailor the size and height of the 2-D or 3-D cell-philic surfaces to a particular application.

The size and height of the cell-philic surfaces will depend on the size of the cell to be captured. In some applications, the cell-philic surfaces can be 1-2 cell diameters of a target cell type to allow for binding of a single cell type. In other applications, cell-philic surfaces can be the large enough to allow for cell division and growth.

In some applications, the cell-philic surfaces can be at least 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30, diameters in width of a target cell type. In some applications, the cell-philic surfaces can be at least 1, 2, 3, 4, 5, 6, 7, 8, 9, 10, 11, 12 ,13 ,14 ,15 ,16 ,17 ,18 ,19, 20, 21, 22, 23, 24, 25, 26, 27, 28, 29,or 30, diameters in height of target cell type. In some applications, the cell-philic surfaces can be a combination of the above- mentioned widths and heights.

The living cells may be grown on the device for a period of time under standard controlled growth conditions use in conventional cell culture, such as controlled temperature, humidity, and gas allowing for a time series and cell linage analysis of the live captured target cell on the device. In some applications the cell growth and division is maintained for at least 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days.

### D. Functionalization of Surfaces

In certain aspects of the disclosure, the cell-philic surfaces can be functionalized with one or more different species. The species used to functionalize a cell-philic surface can include, but are not limited to, chemicals, antibodies, amino acids, peptides, polypeptides, proteins, DNA, RNA, or combinations thereof. The above mentioned functionalization species can be isolated from cells or synthetically made.

The functionalization species can be a binding entity. A binding entity can recognize a target. For example, one non-limiting example of a binding entity is an antibody that recognizes a cell-surface antigen on a target cell. Binding entities can include, but are not limited to, an antibody, an antigen, an aptamer, a nucleic acid (*e.g.* DNA and RNA), a protein (*e.g.* receptor, enzyme, enzyme inhibitor, enzyme substrate, ligand), a peptide, cell-adhesive peptide sequences for example, laminin-derived recognition sequences, such as IKLLI, IKVAV, LRE, PDSGR, RGD, and YIGSR, and the collagen type I sequence, DGEA, and synthetic peptide epitopes, or combinations thereof. One example of a cell-adhesive peptide sequence, includes the RGD peptide sequence which can be used in applications where the recognition and capture of EpCAM expressing cells is desired.

Binding entities to cell cancer markers can also be used with the methods and devices provided herein. Examples of cell cancer markers that can be used include but are not limited to markers for a cancer cells such as breast, prostate, liver, ovary, skin, colon, rectum, cervix, esophagus, stomach, brain, lung, pancreatic, or endometrium cancer. In some applications a cellular marker is epidermal growth factor receptor (EGFR), EpCAM, folic acid receptor, or combinations thereof. In some applications, the cellular marker is E-cadherin, mucin-1, cytokeratin 8 (CK8), cytokeratin 19 (CK19), ERBB2, PDGF, L6, leukocyte associated receptor (LAR), or combinations thereof. In other instances, 2AR, a disintegrin, activator of thyroid and retinoic acid receptor (ACTR), ADAM 11. adipogenesis inhibitory factor (ADIF), alpha 6 integrin subunit, alpha v integrin subunit, alpha-catenin, amplified in breast cancer 1 (AIB1), amplified in breast cancer 3 (AIB3), amplified in breast cancer 4 (AIB4), amyloid precursor protein secretase (APPS), AP-2 gamma, APPS, atp-binding cassette transporter (ABCT), placenta-specific (ABCP), ATP-binding cassette subfamily c member (ABCC1), BAG-1, basigin (BSG), breast cancer estrogen-inducible sequence (BCEI), b-cell differentiation factor (BCDF), B-cell leukemia 2 (BCL-2), B-cell stimulatory factor-2 (BSF-2), BCL-1, BCL-2-associated x protein (BAX), BCRP, beta-1 integrin subunit, beta 3 integrin subunit, beta 5 integrin subunit, beta-2 interferon, beta-catenin, bone sialoprotein (BSP), breast cancer estrogen-inducible sequence (BCEI), breast cancer resistance protein (BCRP), breast cancer type 1 (BRCA1), breast cancer type 2 (BRCA2), breast carcinoma amplified sequence 2 (BCAS2), cadherin, epithelial cadherin-11, cadherin-associated protein, calcitonin receptor (CTR), calcium placental protein (CAPL), calcyclin, calla, CAM5, CAPL, carcinoembryonic antigen (CEA), catenin alpha 1, cathepsin (b, c, d, k, l2, o, o1,v), CD10, CD146, CD147, CD24, CD29, CD44, CD51, CD54, CD61, CD66E, CD82, CD87, CD9, cellular retinol-binding protein 1 (CRBP1), CK7, CK8, CK18, CK19, CK20, claudin-7, c-MET, collagenase-3,common acute lymphocytic leukemia antigen (CALLA), connexin 26 (CX26), connexin 43 (CX43), cortactin, cyclooxygenase-2 (COX-2), CTLA-8,CTR, CTSD, CYCLIN D1, cytokeratin 18, cytokeratin 19,cytokeratin 8,cytotoxic t-lymphocyte-associated serine esterase 8 (CTLA-8), differentiation-inhibiting activity (DIA), DNA amplified in mammary carcinoma 1 (DAM1), DNA topoisomerase ii alpha, DR-NM23, E-cadherin, extracellular matrix metalloproteinase inducer (EMMPRIN), ems1, endothelial cell growth factor (ECGR), platelet-derived (PD-ECGF), enkephalinase, epidermal growth factor receptor (EGFR), episialin, epithelial membrane antigen (EMA), ER-alpha, ER-beta, ERBB2, ERBB4, ERF-1, erythroid-potentiating activity (EPA), ESR1, estrogen receptor-alpha, estrogen receptor-beta, ETS-1, extracellular matrix metalloproteinase inducer (EMMPRIN), fibronectin receptorc beta polypeptide (FNRB), fibronectin receptor beta subunit (FNRB), FLK-1, ga15.3, ga733.2, galectin-3, gamma-catenin, gap junction protein, gap junction protein alpha-1 (GJA1), gap junction protein beta-2 (GJB2), GCP1, gelatinase a, gelatinase b, gliostatin, glucocorticoid receptor interacting protein 1 (GRIP 1), glutathione s-transferase pi (GSTP1), granulocyte chemotactic protein 1 (GCP1), granulocyte-macrophage-colony stimulating factor (GM-CSF), growth factor receptor bound-7 (GRB-7), GSTP, HAP, heat-shock cognate protein 70 (HSC70), heat-stable antigen, hepatocyte growth factor (HGF), hepatocyte growth factor receptor (HGFR), hepatocyte-stimulating factor iii (HSF III), HER-2, hermes antigen, HET, humoral hypercalcemia of malignancy (HHM), ICERE-1, INT-1, ntercellular adhesion molecule-1 (ICAM-1), interferon-gamma-inducing factor (IGIF), interleukin-1 alpha (IL-1A), interleukin-1 beta (IL-1B), interleukin-11 (IL-11), interleukin-17 (IL-17), interleukin-18 (IL-18), interleukin-6 (IL-6), interleukin-8 (IL-8), inversely correlated with estrogen receptor, expression-1 (icere-1), KAI 1, KDR, keratin 8, keratin 18, keratin 19, kiss-1, leukemia inhibitory factor (LIF), lost in inflammatory breast cancer (LIBC), lot ("lost on transformation"), lymphocyte homing receptor, macrophage-colony stimulating factor (GMCSF), melanoma antigen, family a (MAGE3), mammaglobin, maspin, MC56, M-CSF, MDC, MDNCF, MDR, melanoma cell adhesion molecule (MCAM), membrane metalloendopeptidase (MME), membrane-associated neutral endopeptidase (NEP), cysteine-rich protein (MDC), metastasin (MTS-1), metastatic lymph node 64 (MLN64), MMP1, MMP2, MMP3, MMP7, MMP9, MMP11, MMP13, MMP14, MMP15, MMP16, MMP17, moesin (MSN), monocyte arginine-serpin, monocyte-derived neutrophil chemotactic factor, monocyte-derived plasminogen activator inhibitor, MTS-1, MUC-1, MUC18, mucin like cancer associated antigen (MCA), mucin, multidrug resistance protein 1 (MDR, MDR1), multidrug resistance related protein-1 (MRP, MRP-1), N-cadherin, NEP, NEU, neutral endopeptidase, neutrophil-activating peptide 1 (NAP1), nonmetastatic protein 23, homolog 1 (NM23-H1), NM23-H2, nonmetastatic cells 1 (NME1), NME2, nuclear receptor coactivator-1 (NCOA-1), nuclear receptor coactivator-2 (NCOA-2), nuclear receptor coactivator-3 (NCOA-3), nucleoside diphosphate kinase a (NDPKA), nucleoside diphosphate kinase b (NDPKB), oncostatin m (OSM), ornithine decarboxylase (ODC), osteoclast differentiation factor (PDF), osteoclast differentiation factor receptor (ODFR), osteonectin (OSN, ON), osteopontin (OPN), oxytocin receptor (OXTR), p27/KIP1, p300/cbp cointegrator associate protein (P/CIP), p53, p9ka, plasminogen activator inhibitor 1 (PAI-1), PAI-2, parathyroid adenomatosis 1 (PRAD1), parathyroid hormone-like hormone (PTHLH), parathyroid hormone-related peptide (PTHRP), P-cadherin, PD-ECGF, PDGF-B, peanut-reactive urinary mucin (PUM), p-glycoprotein (P-GP), prostaglandin-endoperoxide synthase 2 (PTGS2), prolactin-inducible protein (PIP), plakoglobin (PKGB), plasminogen activator inhibitor (PAI11, PAI-2), plasminogen activator tissue-type (PLAT), plasminogen activator urokinase-type (PLAU), platelet glycoprotein IIIa (GP3A), pleomorphic adenoma gene-like 1 (PLAGL1), polymorphic epithelial mucin (PEM), parathyroid adenomatosis 1 (PRAD1), progesterone receptor (PGR), prostaglandin endoperoxide synthase-2, prostaglandin g/h synthase-2, prostaglandin h synthase-2, PS2, PS6K, psoriasin, PTHLH, PTHRP, RAD51, RAD52, RAD54, RAP46, receptor-associated coactivator 3 (RAC3), repressor of estrogen receptor activity (REA), s100 calcium-binding protein (S100A4, S100A6, S100A7), S6K, SART-1, scaffold attachment factor b (SAF-B), scatter factor (SF), secreted phosphoprotein-1 (SPP-1), secreted protein, acidic and rich in cysteine (SPARC), stanniocalcin (STC1, STC2), steroid receptor coactivator (SRC-1, SRC-2, SRC-3), steroid receptor rna activator (SRA), stromelysin-1, stromelysin-3, thymidine phosphorylase (TP), thyroid hormone receptor activator molecule 1(TRAM-1), tight junction protein 1 (TJP1), tissue inhibitor of metalloproteinase (TIMP1, TIMP2, TIMP3,TIMP4), tenascin c (TNC), tissue plasminogen activator (TPA), transcriptional intermediary factor 2 (TIF2), trefoil factor 1 (TFF1), tumor susceptibility gene 101 (TSG101), testis serine protease 1; (TSP1), thrombospondin (TSP1, TSP2), TSP50, tumor cell collagenase stimulating factor (TCSF), tumor-associated epithelial mucin, urokinase (URK), urokinase-type plasminogen activator (UPA), urokinase-type plasminogen activator receptor (UPAR), uvomorulin, vascular endothelial growth factor (VEGF), vascular endothelial growth factor receptor-2 (VEGFR2), vascular endothelial growth factor-a (VEGFA), vascular permeability factor , very late t-cell antigen beta (vla-beta), vimentin, vitronectin receptor alpha polypeptide (VNRA), vitronectin receptor, von willebrand factor (VWF), VPF, wingless-type mmtv integration site family, member 1 (WNT-1), ZAC, zonula occludens-1 (ZO-1), and combinations thereof.

Cells involved in metastatic tumor formation may be capture and analyzed using the methods and devices described herein. Useful markers for metastatic tumor formation that can be used with the methods and devices include but are not limited to CD105, CD106, CD144, and CD146, TEM1, TEM5, TEM8, CD133, or combinations thereof.

These binding entities can be used alone or in combination with other known cell capture features to trigger cell adhesion to a cell-philic surface of the device or alternatively the binding entities can be use to address certain cell types to known cell-philic surface on the device or aid in both functions. In some applications, the binding entities can enable living cells to be reversibly immobilized such that the target cell can be released from a cell-philic surface.

In one non-limiting example, a cell-philic surface can comprise a first antibody and a second antibody wherein the first antibody specifically binds to a first antigen of the target cell or particle and the second antibody specifically binds to a second, different, antigen of the same target cell or particle. Functionalization species, including antibodies, may be conjugated to a functional molecule that allows for further down-stream analysis.

In some applications, the binding entity can be a functional molecule. Examples of a functional molecule include, but are not limited to, biotin, fluorophores, nucleic acid oligomers, his-tag, digoxigenin, FLAG epitope, or polyhistidine.

In some applications the binding entity may be crosslinked within the hydrogel composition. In other applications where the binding entity is bound to the solid support substrate, a covalent bond may be desirable. A covalent bond can established by either by direct chemical reaction of the binding moiety with the solid support or by first activating the solid support or the binding moiety with a suitable reagent to make it possible to link the solid support and the binding moiety. Examples of suitable activating reagents include, but are not limited to, epichlorohydrin, epibromohydrin, allyl-glycidylether; bis-epoxides such as butanedioldiglycidylether; halogen-substituted aliphatic compounds such as di-chloro-propanol, divinyl sulfone; carbonyldiimidazole; aldehydes such as glutaric dialdehyde; quinones; cyanogen bromide; periodates such as sodium-meta- periodate; carbodiimides; chloro-triazines such as cyanuric chloride; sulfonyl chlorides such as tosyl chlorides and tresyl chlorides; N-hydroxy succinimides; 2-fluoro-1- methylpyridinium toluene-4-sulfonates; oxazolones; maleimides; pyridyl disulfides; and hydrazides.

Depending on the type of cell-philic surface used different methods may be preferable to increase capture efficacy for a particular application. For examples, one non-limiting method provided by the disclosure for capturing a live target cell from a biological sample can comprise the steps of: obtaining a hydrogel, wherein said hydrogel is in contact to a solid substrate, a moiety that binds a live target cell, and an inducible agent; crosslinking said hydrogel, thereby forming a two dimensional surface; contacting a live cell to said hydrogel under suitable conditions that allow said moiety to bind to said live target cell; and applying air pressure thereby allowing said live target cell to be captured within said hydrogel.

In another non-limiting example the disclosure provides a method of capturing a live target cell from a biological sample comprising: obtaining a hydrogel, wherein said hydrogel is in contact to a solid substrate, a moiety that binds a live target cell, and an inducible agent; contacting a live target cell to said hydrogel under suitable conditions that allow said moiety to bind to said live target cell; crosslinking said hydrogel, thereby forming a three dimensional surface thereby capturing said live target cell within said hydrogel.

### E. Cell-Phobic Surfaces

The space between the cell-philic sites on the devices provides herein can be comprised of a cell-phobic surface or region that is, a surface or region which is prohibitive to cell binding (**FIG.1**). In other application the cell-phobic surface can comprise the majority of the surface of the device. In some applications the devices and method provide at least 5%, 10%, 15%, 20%, 25%, 30%, 35%, 40%, 45%, 50%, 55% 60%, 65%, 70% ,75%, 80,%, 85%, 90%, 95% of the surface of the device.

Cell phobic regions can comprise one or more types of cell-phobic surfaces. Non-limiting example of materials that can be used to make a cell-phobic surface include any materials which are known to be prohibitive of cell binding and cell adhesion such as, for example, poly(ethylene glycol) (PEG) cell phobic polymers or other cell-phobic nanomaterials known to be prohibitive of cell binding and cell adhesion.

### F. Agents and Activatiable-Release Stimuli

The device can deliver one or more stimuli to the single live captured cell. A stimulus or agent can be an activator, an analyte, an antigen, a therapeutic compound, an inhibitor or a compound capable of impacting a cellular pathway, or environmental cues and inputs from other cells or components of a cellular microenvironment (e.g. extracellular matrix proteins and the like). In some instances, the stimuli of the device can act to simulate the cell extracellularly, intracellularly, or both.

In some applications, the stimuli or agent can be linked the solid substrate of the device. In some applications, the stimuli or agent can be within a hydrogel composition. In some applications, the stimuli or agent can be in crosslinked within a hydrogel composition. In some applications, the stimuli or agent can be linked to an activatiable analyte specific reagent (aASR) that is linked to the solid substrate of the device. In some applications, the stimuli or agent can be linked to an activatiable analyte specific reagent (aASR) that is crosslinked to the hydrogel composition.

The stimulus can be contained at a cell-philic site using an activatiable analyte specific reagent (aASR) **(****FIG. 6A****).** In certain aspects of the disclosure of ASR is contained on a bead. In certain aspects of the disclosure the aASR is combined with the polymer that forms the cell-philic site on a device. The activatiable analyte can be activated by light, temperature, or by chemical treatment depending on the type of analyte used.

In some applications, the aASR can be activated. For example, the aASR can by activated by various means such as by exposure to light, temperature, or by a chemical treatment. Frequently, after activation of the aASR the stimulus is released and available to interact with the single viable cell contained within a cell-philic site. In some applications, the aASR is not capable of being activated. In other applications, the stimulus can be contained at a cell-philic site using an activatiable nanoparticle. The activatiable nanoparticle can be activated by light, temperature, or by chemical treatment depending on the type of nanoparticle used.

In some applications, a stimulus of the device is of known concentration and can be attached to the device substrate or in the surface of a cell-philic surface. In some applications, a stimulus of the device is of unknown concentration and can be attached to the device substrate or in the surface of a cell-philic surface.

The stimulus can increase or decrease the activity of a cellular signaling pathway such that is yields a cellular response. In some applications, the stimulus can cause cell death, cause senescence, promote growth, change cell morphology or promote cell division of the live target cell on the device. In some applications, the stimulus may not change the status of the cell. In some applications, the stimulus will have a known effect on one or more control cells and thereby function as a positive response control. In some applications, the stimulus is known to not change the status of certain cell types and the stimulus can be used as a negative response control.

The stimulus can be activatiable, that is, the stimulus can be in a quiescent state (*e.g.* an inactive form or tethered to a substrate such that it is prohibited from interacting with a cell) until it is induced to an activated state capable of inciting a response in a cell. Once activated, the stimulus can be released and allowed to interact with a cell. One non-limiting example includes, where the stimulus of known concentration is a pro-drug such that it can be cleaved or transformed into an active form and released into a single cell at a particular time point determined by the operator of the device. In another non-limiting example the stimulus can be bound to the device. In some applications, the stimulus can be encapsulated in form of an activatiable bead such that the bead can be induced to be opened and release it contents to a single live cell on the device at a particular time point chosen by the operator.

In some applications, the concentration of the stimulus and the time at which the stimulus is activated or released can be determined precisely by the operator of the device. In some applications, the concentration of the stimulus and the time at which the stimulus is activated or released can be determined by the rate of diffusion.

In certain aspects of the disclosure it is contemplated that one or more different stimuli of known concentration it located in one cell-philic site, such that a combination of different stimuli can be delivered to a single cell on the device. In certain aspects it is contemplated that there are many different types of stimuli bound to the device at known addressable cell-philic site locations on the device. In certain aspects of the disclosure it is contemplated there are many different concentrations of the stimulus bound to the device at known addressable cell-philic site locations on the device, such that at dose-dependent response can be determined from the device.

In some applications, the stimulus or agent can be a chemotherapeutic. Chemotherapeutics are pharmacological compounds that are known to be used in the treatment of cancer or suspected in being useful in the treatment of cancer. Types of chemotherapeutics that can be used as a stimulus can include: alkylating agents, anthracyclines, cytoskeletal disruptors, epothilones, histone deacetylase inhibitors, inhibitors of topoisomerase I, inhibitors of topoisomerase II, kinase inhibitors, monoclonal antibodies, nucleotide analogs (and precursor analogs), peptide antibiotics, platinum-based agents, retinoids, vinca alkaloids (and derivatives), or combinations thereof. Some non-limiting examples of chemotherepeutic agents include: actinomycin, retinoic acid, azacitidine, azathioprine, BCNU, bleomycin, bortezomib, carboplatin, capecitabine, CCNU, cisplatin, ciglitazone ("CGZ"), chlorambucil, cyclophosphamide, cytarabine, daunorubicin, docetaxel, doxifluridine, doxorubicin, epirubicin, epothilone, etoposide, fluorouracil, gemcitabine, hydroxyurea, idarubicin, imatinib, irinotecan, isotretinoin, mechlorethamine, mercaptopurine, methotrexate, mitoxantrone, oxaliplatin, paclitaxel, pemetrexed, pioglitazone ("PGZ"), procarbazine, rosiglitazone ("RGZ"), teniposide, ternozolomide, thalidomide, tioguanine, topotecan, troglitazone ("TGZ"), tumor necrosis factor-related apoptosis-inducing ligand ("TRAIL"), valrubicin, vinblastine, vincristine, vindesine, vinorelbine,VP-16, or combinations thereof.

In some applications, the stimuli or agent include chemical and biological entities, physical or environmental stimuli. Chemical and biological stimuli that can be used with the methods and devices provided herein include, but are not limited to, growth factors, mitogens, cytokines, drugs, immune stimuli, ions, neurotransmitters, adhesion molecules, hormones, small molecules, inorganic compounds, polynucleotides, antibodies, natural compounds, lectins, lactones, chemotherapeutic agents, biological response modifiers, carbohydrate, proteases, free radicals, or combinations thereof. Stimuli used with the methods and devices provided herein can also include complex and undefined biologic compositions that may comprise cellular or botanical extracts, cellular or glandular secretions, physiologic fluids such as serum, amniotic fluid, venom or combinations with the above stimuli. Physical and environmental stimuli that can be used include but are not limited to, electromagnetic, ultraviolet, infrared or particulate radiation, redox potential, pH, the presence or absences of nutrients, changes in temperature, changes in oxygen partial pressure, changes in ion concentrations, the application of oxidative stress or combinations thereof.

In some applications, different stimuli can be used at the plurality of cell-philic sites. Different stimuli can include, but are not limited to, one type of stimuli at different concentrations, a plurality of different stimuli, plurality of different stimuli at different concentrations, or combinations thereof. In some applications, one or more cell-philic sites of the deivce will contain no stimulus, a control type stimulus or both.

In some applications, stimulation of a single cell can include exposing a single cell to more than one stimulus. For example, a single live captured cell of the device can be exposed to at least 2, 3, 4, 5, 6, 7, 8, 9, 10, 11, 12, 13, 14, 15, 16, 17, 18, 19, 20 or more stimuli.

### G. Microfluidic Channels and Chambers

The device can have one or more channels through which regents can be administered to the capture cell on the device. Reagents can include, but are not limited to, nutritional media, cell markers, dyes, molecular barcodes, antibodies, agents, stimulus, buffers, water, sugars, chemicals, gas, therapeutic agents or combinations thereof. The cell capture array can comprise at least one cell-philic site intersected by, or connected to, a channel which can be further comprises of a controllable valves which allows the microfluidic channel to open or closed by the operator of the deivce. The microfluidic channel may contain analytical components appropriate to conduct a desired analysis on the device. The dimensions of the channels can have an impact on the performance of the cell capture array and one skilled in the art can make any necessary modifications to achieve optimal performance.

The microfluidic channels may be substantially identical to each other in terms of dimensions, material, analytical component(s), such that cells captured in different cell-philic sites are separately subjected to the same treatment and analysis as each other, allowing direct comparison of results.

The microfluidic channels of the device may be many arrangements. The channels may be parallel or perpendicular to each other. In an alternative arrangement, channels may radiate from a central point. Parallel channels can also be arranged to extend in different directions from a central point.

In some applications, a liquid or reagent may travel through the microfluidic channels through a variety of means. Movement of the liquid or reagent may be passive, for example by diffusion, osmosis, capillary action, or due to the effect of gravity. Wicking may be used to draw water through the support from a reservoir. In some applications, the movement of the liquid or reagents through the device may be by active means, for example by pumping, applying air pressure, or by electrokinetic means.

### H. Lid Gasket

The gasket or lid may perform a number of functions in the device. One function of the lid is to effectively seal the array, thereby forming one or more domain chambers comprising one or more cell-philic sites and/or cell-phobic sites. In doing so, the lid can receive the contents of the cell-philic sites while maintaining the spatial arrangement and separation of the domains, and the contents of the cell-philic sites. In another application the lid seals the array, thereby forming one or more wells of the device comprising one or more cell-philic sites and/or cell-phobic sites.

In a simple form, the lid may be a glass slide or plastic membrane. In some applications, the lid may be more complex, that is the lid is further modified such that is functionalized with cell binding moiety, cell nutrients, reagents, buffers, chemicals, therapeutic agents, or combinations thereof.

In certain applications, the lid gasket is comprised of inlet and outlet ports. In this configuration the device can allow for continuous flow of fluids across the device surfaces. In certain aspects of the disclosure the lid gasket has inlet and outlet ports to allow the flowing of the single cell suspension, reagents, stimuli or whole blood to contact the cell-philic surfaces on the array **(****FIG. 4****).** In certain applications, the lid gasket is comprised of inlet and outlet ports and is further modified such that is functionalized with cell binding moiety, cell nutrients, reagents, buffers, chemicals, therapeutic agents, or combinations thereof.

In other aspect the ability to continuous flow of fluids can be used to supplement the cells with life-supporting media, buffers, or cell labeling regents. In yet other aspects, the inlet and outlet ports can provide a means to allow the surfaces to be washed by buffers, chemicals, water and the like to remove unbound or non-specifically bound particles such as, cells, dyes, antibodies, cellular debris, or excess or unbound stimuli.

In some applications, the lid gasket of the device does not have inlet and outlet ports.

### V. METHODS

### A. Single Cell Analysis Methods

The devices and methods provided by the present disclosure allow for the analysis and prediction of how an individual cell, in a heterogeneous or homogenous population, will react to a given stimulus or therapeutic agent.
In some applications, the single captured cells can be subjected to cell signaling analysis by detecting the presence of the activation of a signaling protein. In some applications, cell response may be measured at about 5 min, 10 min, 15 min, 20 min, 25 min, 30 min, 35 min, 40 min, 45 min, 50 min, 55 min, 1 hr, 2 hr, 3 hr, 4 hr, 5 hr, 6 hr, 7 hr, 8 hr, 9 hr, 10 hr, 11 hr, 12 hr, 13 hr, 14 hr, 15 hr, 16 hr, 17 hr, 18 hr, 19 hr, 20 hr, 21 hr, 22 hr, 23 hr, 24 hr, 30 hr, 36 hr, 40 hr, 48 hr, 72 hr, or more after a cell is contacted by an agent or stimuli.

The detection of the activation of a signaling protein can be achieved by antibodies that recognized changes or modifications of proteins (e.g. activation state-specific antibody) such as the phosphorylation modification of a particular amino acid on a signaling protein. Other molecule hallmarks of protein activation that can be used with the methods and devices provide herein are chemical additions or modifications such as glycosylation, acetylation, methylation, biotinylation, glutamylation, glycylation, hydroxylation, isomerization, prenylation, myristoylation, lipoylation, phosphopantetheinylation, sulfation, ISGylation, nitrosylation, palmitoylation, SUMOylation, ubiquitination, neddylation, citrullination, amidation, and disulfide bond formation, disulfide bond reduction or combinations thereof. Other possible chemical additions or modifications of biomolecules include the formation of protein carbonyls, direct modifications of protein side chains, such as o-tyrosine, chloro-, nitrotyrosine, and dityrosine, and protein adducts derived from reactions with carbohydrate and lipid derivatives.

In some applications, the cell singling of an captured cell can be detected off the device by downstream applications such as, flow cytometry, mass spectrometry, radioimmunoassay (RIA), enzyme linked immunoabsorbance assay (ELISA), immunohistochemistry, immunofluorescent histochemistry, reversed phase assays, homogeneous enzyme immunoassays, and related non-enzymatic techniques, Western blots, Far Western, Northern Blot, Southern blot, whole cell staining, immunoelectronmicroscopy, PCR, gene array, protein array, mass spectrometry, nucleic acid sequencing, next generation sequencing, patch clamp, 2-dimensional gel electrophoresis, differential display gel electrophoresis, microsphere-based multiplex protein assays, label-free cellular assays, or a combinations thereof.

In some applications, the individual captured cell can be analyzed to see if treatment with a differentiating agent has pushed a cell type along a specific tissue lineage to terminally differentiated path with subsequent loss of proliferative or renewal capacity. Such analysis may be used determine the efficacy of a leukemia treatment which aims to keep the number of dedifferentiated cells associated with disease at a low level, thereby preventing the development of an overt leukemic state. In another application, such cell linage analysis studies provided by the methods and devices of the present disclosure the stand to facilitate studies in regenerative tissue medicine to determine which stimuli and agents are effective at directing pluripotent or multipotent stem cells down a desired tissue or organ specific lineage.

The disclosure provides methods and devices for on-array analysis of enumerating a cell of interest in a heterogeneous biological sample, phenotyping, morphology, genotyping, intracellular cell signaling analysis, cell-cell or tissue system analysis with or with the use of computational methods.

The disclosure also provides devices and methods off-array analysis, such that a single cell capture can be released from the array and then collected and analyzed by other devices designed for cellular analysis such as cell counting, phenotyping, genotyping mutational analysis, intracellular cell signaling analysis, expression analysis, DNA and RNA sequencing, and tissue system analysis with or without computational methods.

In some applications, proteins or glycoproteins, including peptides and amino acids, can be obtained from the captured single cells can be subjected to, for example, but not limited to, amino acid or peptide analysis, sequencing, gas chromatography-mass spectrometry, liquid phase mass spectrometry and other techniques known to those skilled in the art of protein analyses.

In some applications, the captured cells on the device can be analyzed for morphology, surface protein expression, internal cellular structures, viral or microorganism infection by the used of light microscopy, electron microscopy, scanning microscopy, or by using biosensors or a combination thereof.

A variety of cellular morphological characteristics may be measured using any of the above techniques, such as pleomorphisms, adhesion, migration, binding, division status, or other structural cell characteristics. In addition, the methods and devices provided herein allow for the analysis the cell size distribution in a heterogeneous sample.

The captured cells may be labeled with fluorescent markers such as nucleic acid dyes or fluorescently labeled antibodies while attached and visualized by fluorescent microscopy or by measuring a fluorescent signal by high content cell screening. A variety of cellular characteristics may be measured using any of the above techniques, such as, for example the level of expression of a cell marker, phosphorylation, protein glycosylation, DNA methylation or a combination thereof.

In some applications, genome and proteome analysis can be used with the methods and devices of the disclosure. Examples of genomic and proteomic methods that can be used include but are not limited to, DNA or RNA analysis, mRNA, microRNA, proteome analysis, cell surface markers, or metabolome analysis. For example, genomic studies on the presence of a particular sequence or mutation (*e.g*., mutational status of DNA), deletion (under-expression mRNA or RNA), duplication (over-expression mRNA or RNA), rearrangement, insertion in DNA, RNA, or the over-expression or under-expresion or mutational status of microRNA may be detected, and used to determine the disease status, prognosis, diagnosis, or the likelihood of drug response in a subject or the risk or likelihood of presenting with a disease in the future. Furthermore, the above-mention genomic analysis may be combined with other cell analysis to determine disease status, prognosis, diagnosis, or the likelihood of drug response in a subject or the risk or likelihood of presenting with a disease in the future.

Cellular analysis using the methods and devices of the disclosure may further include the genomic and proteomic analysis of mitochondrial DNA, telomerase, or nuclear matrix proteins in a capture cell (for mitochondrial mutations in cancer, see, Parrella et al., Cancer Res. 61:7623-7626 (2001), Jones et al., Cancer Res. 61:1299-1304 (2001), and Fliss et al., Science 287:2017-2019 (2000); for telomerase, see, e.g., Soria et al., Clin. Cancer Res. 5:971-975 (1999)).

In some applications of the methods and devices provide herein, the single captured cells can be subjected to *in situ* hybridization analysis, such as FISH for detection of aneuploidy or other chromosomal features or to determine the tissue or tissues of origin of the cells being analyzed.

### B. Cell Phenotyping and Enumeration Methods

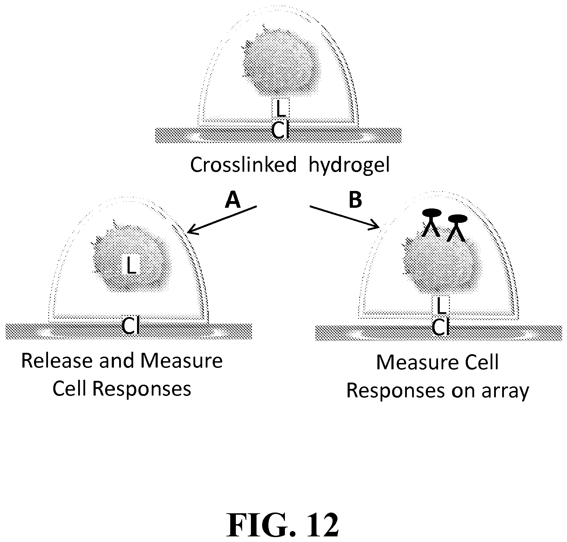

In one aspect the disclosure provides methods and devices for on-array single cell capture and phenotyping. In another one aspect the disclosure provides a method and device for off-array single cell capture and phenotyping **(****FIG. 12****).**

Provided herein are methods for classifying a cell's phenotype, cell type, or cellular response. Cell phenotyping can be determined by absence or presence of cell surface markers, intracellular markers, cell signaling response to a stimulus, or combinations thereof. In some applications of the method, additional cellular elements or feature can be used to further classify a cell to a phenotype, such as the expression level of extracellular or intracellular markers, nuclear antigens, enzymatic activity, protein expression and localization, cell cycle analysis, epithelial-mesenchymal transition (EMT) status, chromosomal analysis, cell volume, and morphological characteristics like granularity and size of nucleus or other distinguishing characteristics of cell morphology.

In another one aspect the disclosure provides a method and device for on-array single cell capture, phenotyping and enumeration. In another one aspect the disclosure provides a method and device for off-array single cell capture, phenotyping, and enumeration.

In another one aspect the disclosure provides methods and devices for on-array single cell capture, phenotyping, and enumeration determines a particular disease or disease subtype for example, various subtypes of breast cancer, aggressive or non-aggressive cancer, or metastatic or non-metastatic cancer. In another one aspect the disclosure provides a method and device for off-array single cell capture, phenotyping, and cell enumeration determines a particular disease or disease subtype for example, various subtypes of breast cancer, aggressive or non-aggressive cancer, or metastatic or non-metastatic cancer.

In another one aspect the disclosure provides a method for on-array single cell capture, phenotyping and cell enumeration determines a circulating tumor cell (CTC) cell. In another one aspect the disclosure provides a method and device for off-array single cell capture, phenotyping, and cell enumeration of a CTC cell.

### C. System Biology Methods

In some application the devices and methods can be applied to a cell system. For example, a cell system can include, but is not limited to, more than one cells types, an whole or portion of an intact organ, a tissue slice or a portion from a tissue, a circulating tumor cell in blood, or the like.

The methods and devices provide for cell system analysis measuring various features of the cell system such as protein status, genomic status such as DNA and RNA expression, proteomics, and their cell signaling responses to agent, environmental stimuli such as cell-cell contacts to characterize a living systems and complex diseases by the aid of computational analysis.

It one aspect the disclosure provides devices and methods for collecting data of a cell system regarding phenotype, phenotype abundance, environment heterogeneity, signaling pathways or transcriptome (*e.g*. expressed mRNA), comprising the steps of collecting single cell analysis data using any of the methods described herein and sending said data to a computer that is capable of performing computational analysis.

Some exemplary cell signaling proteins that can be assayed in a cell system or a captured single cell include, but are not limited to, kinases, HER receptors, PDGF receptors, Kit receptor, FGF receptors, Eph receptors, Trk receptors, IGF receptors, Insulin receptor, Met receptor, Ret, VEGF receptors, TIE1, TIE2, FAK, Jak1, Jak2, Jak3, Tyk2, Src, Lyn, Fyn, Lck, Fgr, Yes, Csk, Abl, Btk, ZAP70, Syk, IRAKs, cRaf, ARaf, BRAF, Mos, Lim kinase, ILK, Tpl, ALK, TGF-β receptors, BMP receptors, casein kinases, PDK1, SGK1, SGK2, SGK3, Akt1, Akt2, Akt3, p90Rsks, p70S6Kinase, MEKKs, ASK, MLKs, DLK, PAKs, Mek 1, Mek 2, MKK3/6, MKK4/7, ASK1,Cot, NIK, Bub, Myt 1, Weel, Prks, PKCs, PKAs, ROCK1, ROCK2, Auroras, CaMKs, MNKs, AMPKs, MELK, MARKs, Chk1, Chk2, LKB-1, MAPKAPKs, Pim1, Pim2, Pim3, IKKs, Cdks, Jnks, Erks, Erk1, Erk2, IKKs, GSK3α, GSK3β, Cdks, CLKs, PKR, PI3Kinase class 1, class 2, class 3, mTor, SAPK/JNK1,2,3, p38s, PKR, DNA-PK, ATM, ATR, phosphatases, SHPs, MAP kinase phosphatases (MKPs), Dual Specificity phosphatases (DUSPs), CDC25 phosphatases, Tyrosine phosphatase, Eyes absent (EYA) tyrosine phosphatases, Receptor protein tyrosine phosphatases (RPTPs), LAR phosphatase, CD45, Non receptor tyrosine phosphatases (NPRTPs), Slingshot phosphatases (SSH), serine phosphatases, PP2A, PP2B, PP2C, PP1, PP5, inositol phosphatases, PTEN, SHIPs, myotubularins, lipid signaling, phosphoinositide kinases, phopsholipases, prostaglandin synthases, 5-lipoxygenase, sphingosine kinases, sphingomyelinases, adaptor/scaffold proteins, Shc, Grb2, BLNK, LAT, SLAP, Dok, KSR, MyD88, Crk, CrkL, GAD, Nck, Grb2 associated binder (GAB), Fas associated death domain (FADD), TRADD, TRAF2, RIP, T-cell leukemia family, cytokines, IL-2, IL-4, IL-8, IL-6, interferon γ, interferon α, cytokine regulators, suppressors of cytokine signaling (SOCs), ubiquitination enzymes, Cbl, SCF ubiquitination ligase complex, APC/C, adhesion molecules, integrins, Immunoglobulin-like adhesion molecules, selectins, cadherins, catenins, focal adhesion kinase, p130CAS, cytoskeletal/contractile proteins, fodrin, actin, paxillin, myosin, myosin binding proteins, tubulin, eg5/KSP, CENPs, heterotrimeric G-proteins such as guanine nucleotide exchange factors and small molecular weight GTPases, β-adrenergic receptors, muscarinic receptors, adenylyl cyclase receptors, H-Ras, K- Ras, N-Ras, Ran, Rac, Rho, Cdc42, Arfs, RABs, RHEB, Vav, Tiam, Sos, Dbl, PRK, TSC1,2, GTPase activating proteins, Ras-GAP, Arf-GAPs, Rho-GAPs, regulators of translation, pS6, 4EPB-1, eIF4E-binding protein, regulators of transcription, RNA polymerase, initiation factors, and elongation factors, cell cycle regulators, Cdk4, Cdk 6, Cdk 2, Cdk1, Cdk 7, Cyclin D, Cyclin E, Cyclin A, Cyclin B, Rb, p16, p14Arf, p27KIP, p21CIP, proteins involved in apoptosis such as caspases, Caspase 2, Caspase 3, Caspase 6, Caspase 7, Caspase 8, Caspase 9, Bc1-2, Mc1-1, Bc1-XL, Bc1-w, Bc1-B, A1, Bax, Bak, Bok, Bik, Bad, Bid, Bim, Bmf, Hrk, Noxa, Puma, IAPs, XIAP, Smac, Ets, Elk, SMADs, Rel-A (p65-NFKB), CREB, NFAT, ATF-2, AFT, Myc, Fos, Sp1, Egr-1,T-bet, HIFs, FOXOs, E2Fs, SRFs, TCFs, Egr-1, β-catenin, FOXO, STAT1, STAT3, STAT4, STAT5, STAT6, p53, WT-1, HMGA molecular chaperones, Hsp90s, Hsp70, Hsp27, vesicular protein sorting (Vsps), hydroxylases, prolyl-hydroxylases PHD-1, 2 and 3, asparagine hydroxylase FIH transferases, isomerases, Pin1 prolyl isomerase, topoisomerases, deacetylases, Histone deacetylases, sirtuins, acetylases, histone acetylases, CBP/P300 family, MYST family, ATF2, methylases, DNA methyl transferases, demethylases, Histone H3K4 demethylases, H3K27, JHDM2A, UTX, tumor suppressor genes, VHL, WT-1, p53, Hdm, PTEN, proteases, ubiquitin proteases, urokinase-type plasminogen activator (uPA) and uPA receptor (uPAR) system, cathepsins, metalloproteinases, esterases, hydrolases, separase, ion channels, potassium channels, sodium channels, molecular transporters, multi-drug resistance proteins, P-Gycoprotein, nucleoside transporters, transcription factors/ DNA binding proteins, metabolic enzymes, acetyl-CoA carboxylase, ATP citrate lyase, nitric oxide synthase, vesicular transport proteins, caveolins, endosomal sorting complex required for transport (ESCRT) proteins, or combinations thereof.

The methods and devices provide for cell system analysis of the cell proteome. The cell proteome is defined as the totality of the proteins present in a sample such as a, tissue, organism, or cell culture. Proteomics includes the analysis of global changes of protein expression in a sample under certain conditions such as disease or treatment with therapeutic agents. Proteomics typically includes identification of the individual proteins and analysis of the data using bioinformatics. Proteomics methods are valuable supplements to other methods of gene expression profiling, and can be used, alone or in combination with other methods of the present disclosure.

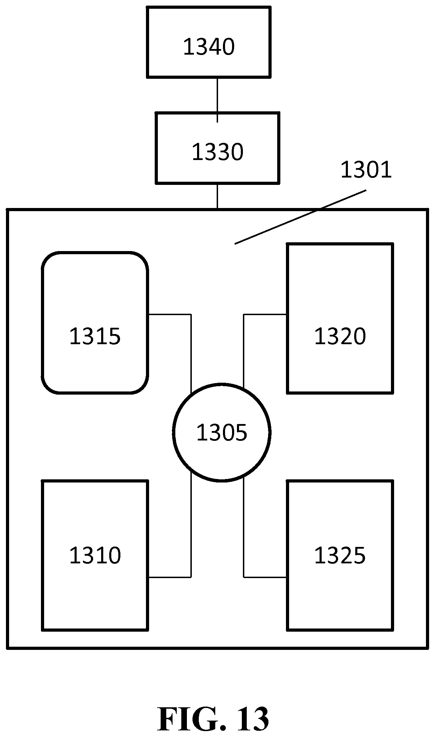

Particularly when the system biology method is employed, often a computer can be connected to a laboratory instrumentations required for cell analysis methods provided herein such as, optical instrumentation, biosensors, DNA or RNA sequencing instrumentation. Data corresponding to the analysis of the cell system can further be stored, for example the data can be stored on a computer-readable medium which can be extracted from the computer to perform computational analysis. Data can be transmitted from the computer to a remote location, for example, via the internet to perform computational analysis on the collected cell system data using a computer system such as the one illustrated in **FIG.13****,** or variations thereof.

### D. Therapeutic Response Methods

One aspect the disclosure provides devices and methods for determining likelihood of a response by a subject to a therapeutic agent comprising: obtaining a biological sample from a subject; binding cells from the biological sample to the device; exposing a cell to a stimulus; performing a single cell analysis to detect response from the cell; and using said detected response to determine the likelihood of a response by a subject to a therapeutic agent.

One aspect the disclosure provides devices and methods for identifying a signaling pathway utilized by a disease-state cell, comprising: obtaining a cellular sample from a subject; binding said cells to the device; exposing said cell to a stimulus; performing a cell analysis; using said cell analysis to identify at least one disease-state cell by comparing the said cell analysis from the at least one disease-state cell to a second cell analysis from a non-disease state cell or known responder cell, thereby identifying a signaling pathway utilized by a disease-state cell.

It one aspect the disclosure provides device and method for diagnosing a subject with a condition comprising: obtaining a cellular sample from a subject, binding said cells to the device, exposing said cell to a stimulus, performing a cell analysis, using said cell analysis to identify at least one disease-state cell by comparing the said cell analysis from the at least one disease-state cell to a second cell analysis from a non-disease state cell, thereby diagnosing the presence or absence of a condition associated with the disease-state cell in a subject.

### E. Drug Screening Methods

One aspect the disclosure provides devices and methods for screening a therapeutic agent for its efficacy for treating a particular disease. In another aspect the disclosure provides devices and methods for screening a diseased cell for sensitivity to a therapeutic agent. In another aspect the disclosure provides devices and methods for screening a diseased cell for resistance to a therapeutic agent.

The present disclosure also provides devices and methods for the development, identification of new therapeutic agents, or refining of the performance of pre-existing therapeutic agents (e.g., second generation drugs or drug repurposing). For example, it is contemplated that the devices provided herein can be used to isolate a target disease cell or populations of the target disease cell and further characterize its signaling response, cell surface marker expression, and DNA or RNA expression and sequence to aid the development of therapeutic agents to new potential targets discovered by the provide devices and methods. Potential therapeutic targets characterized from the methods and devices provided by the disclosure include, without limitation, particular dysregulated genes or proteins and dysregulated signaling pathways as compared to non-disease or normal state cells.

The device of the present disclosure can be used in high-throughput drug screening methods. Such methods can include, using the cell capture array device and the agent transfer device to transfer the drug to the cells as shown in **FIG. 8****.** A high-throughput drug screening method can comprise one or more of the following steps: (a) cells in suspension are added to the cell-philic surface on a cell capture array **FIG. 8A****;** (b) cells are then localized to the cell-philic regions on said array; (c) cells are then immobilized into a hydrogel or hydrogel-like composition; (d) an agent transfer device loaded with stimuli such as therapeutic drugs, ligands, antibodies, siRNAs or the like are dispensed onto the surface of said agent transfer device thereby creating an agent transfer device; **FIG. 8B****.** The stimuli of the agent transfer device can be comprise of one or many different type of stimuli. The dispensed to the agent transfer device can be of known concentration, unknown concentration, one concentration or various different concentrations. Finally, in step (e) the stimuli is delivered to the first cell capture array by "mating" or contacting the cell capture array to the agent transfer device such that the cell-philic surfaces of the cell capture array and the surface of the agent transfer device are allowed to touch or contact one another, thereby facilitating the transfer of the stimuli from the agent transfer device to the captured cell on the cell capture array **FIG. 12C****.**

In some applications the devices and methods provide at least 5%, 10%, 15%, 20%, 25%, 30%, 35%, 40%, 45%, 50%, 55% 60%, 65%, 70% ,75%, 80,%, 85%, 90%, or 95% more accurate drug response results than drug screening methods using standard adherent, two-dimensional cultures. In some applications the devices and methods provide at least 5%, 10%, 15%, 20%, 25%, 30%, 35%, 40%, 45%, 50%, 55% 60%, 65%, 70% ,75%, 80,%, 85%, 90%, or 95% similar drug response results as *in vivo* pre-clinical testing. In some applications the devices and methods provide at least 5%, 10%, 15%, 20%, 25%, 30%, 35%, 40%, 45%, 50%, 55% 60%, 65%, 70% ,75%, 80,%, 85%, 90%, or 95% similar drug response results as clinical testing.

### F. Prognostic and Diagnostic Methods

The systems, devices, and methods of the disclosure provide for the diagnosis and prognosis of a disease. Furthermore, devices and methods of the disclosure are useful in assessing the prevention of unnecessary therapy that could result in harmful side-effects by providing pre-treatment studies or follow-up studies on a biological sample from an individual undergoing or suggested for treatment.

Frequently, the present disclosure can be used with circulating tumor cells, tumor biopsies, for prognosis and diagnosis of any of a wide variety of cancers including, without limitation, solid tumors and leukemia's including but not limited to, apudoma, choristoma, branchioma, malignant carcinoid syndrome, carcinoid heart disease, carcinoma (*i.e.* Walker, basal cell, basosquamous, Brown-Pearce, ductal, Ehrlich tumor, Krebs 2, merkel cell, mucinous, non-small cell lung, oat cell, papillary, scirrhous, bronchiolar, bronchogenic, squamous cell, and transitional cell), histiocytic disorders, leukemia (*i.e*. B-cell, mixed-cell, null-cell, T-cell, T-cell chronic, HTLV-11-associated, lymphocytic acute, lymphocytic chronic, mast-cell, and myeloid), histiocytosis malignant, Hodgkin's disease, immunoproliferative small, non-Hodgkin's lymphoma, plasmacytolma, reticuloendotheliosis, melanoma, chondroblastoma, chondroma, chondrosarcoma, fibroma, fibrosarcoma, giant cell tumors, histiocytoma, lipoma, liposarcoma, mesothelioma, myxoma, myxosarcoma, osteoma, osteosarcoma, Ewing's sarcoma, synovioma, adenofibroma, adenolymphoma, carcinosarcoma, chordoma, craniopharyngioma, dysgerminoma, hamartoma, mesenchymoma, mesonephroma, myosarcoma, ameloblastoma, cementoma, odontoma, teratoma, thymoma, trophoblastic tumor, adenocarcinoma, adenoma, cholangioma, cholesteatoma, cylindroma, cystadenocarcinoma, cystadenoma, granulose cell tumor, gynandroblastoma, hepatoma, hidradenoma, islet cell tumor, icydig cell tumor, papilloma, sertoli cell tumor, theca cell tumor, leiomyoma, leiomyosarcoma, myoblastoma, myoma, myosarcoma, rhabdomyoma, rhabdomyosarcoma, ependymoma, ganglioneuroma, glioma, medulloblastoma, meningioma, neurilemmoma, neuroblastoma, neuroepithelioma, neurofibroma, neuroma, paraganglioma, paraganglioma nonchromaffin, angiokeratoma, angiolymphoid hyperplasia with eosinophillia, angioma sclerosing, angiomatosis, glomangioma, hemangioendothelioma, hemangioma, hemangiopericytoma, hemangiosarcoma, lymphangioma, lymphangiomyoma, lymphangiosarcoma, pinealoma, carcinosarcoma, chondroscarcoma, cystosarcoma, phyllodes, fibrosarcoma, hemangiosarcoma, leiomyosarcoma, leukosarcoma, liposarcoma, lymphangiosarcoma, myoswarcoma, my osarcoma, ovarian carcinoma, rhabdomyosarcoma, sarcoma (i.e. Ewing's experimental, Kaposi's and mast-cell), neoplasms (*i.e.,* bone, breast, digestive system, colorectal, liver, pancreatic, pituitary, testicular, orbital, head and neck, central nervous system, acoustic, pelvic, respiratory tract, and urogenital, neurofibromatosis, and cervical dysplasia.

The systems, devices, and methods of the disclosure can also be used for the characterization and detection of biomarkers. Biomarkers are uniquely expressed biomolecules on a diseased cell and have been viewed as key targets for guiding the development of diagnostic tests and drug development strategies. Thus, the methods and devices disclosed herein allow for determining new biomarkers, which are associated with a particular cell type or a cell population or a cell subpopulation that define a healthy state, a person at risk for disease, or a person who will likely be responsive to a particular therapeutic or therapeutic regimen.

A molecular signature for a condition or diseased is often referred to as a "profile". Generally, a profile is defined by one or more biomolecules in a single cell or a subset of cell populations whose presence indicates the status or prediction of a disease. A signature or profile can comprise one or more biomolecules from at least 5 biomolecules, at least 10 biomolecules, at least 15 biomolecules, at least 20 biomolecules, at least 25 biomolecules, at least 50 biomolecules, at least 75 biomolecules, at least 100 biomolecules, at least 150 biomolecules, at least 200 biomolecules, at least 300 biomolecules, at least 400 biomolecules, at least 500 biomolecules, or more biomolecules derived from a single or subset of cell populations. Thus, the methods and devices disclosed herein allow for determining profiles, which are associated with a particular cell type or a cell population or a cell subpopulation that define a healthy state, a person at risk for disease, or a person who will likely be responsive to a particular therapeutic or therapeutic regimen.

It is contemplated that a signature or profile derived from the method and device of the present disclosure could be used to determine the presence or absence of a condition or disease. It is contemplated that a signature or profile derived from the method and device of the present disclosure could be used to determine the severity of a condition or disease. It is contemplated that a signature or profile derived from the method and device of the present disclosure could be used to determine the response of a disease to a therapeutic agent. It is contemplated that a signature or profile derived from the method and device of the present disclosure could be used to determine which therapeutic options would be most productive in the treatment of a disease and given to a health-care professional by means of a report for clinical management.

In some applications the devices and methods provide at least 5%, 10%, 15%, 20%, 25%, 30%, 35%, 40%, 45%, 50%, 55% 60%, 65%, 70% ,75%, 80,%, 85%, 90%, or 95% prognosis accuracy. In some applications the devices and methods provide at least 5%, 10%, 15%, 20%, 25%, 30%, 35%, 40%, 45%, 50%, 55% 60%, 65%, 70% ,75%, 80,%, 85%, 90%, or 95% diagnosis accuracy. In some applications the devices and methods provide at least 5%, 10%, 15%, 20%, 25%, 30%, 35%, 40%, 45%, 50%, 55% 60%, 65%, 70% ,75%, 80,%, 85%, 90%, or 95% drug response accuracy.

### G. Clinical Management Methods

The health-care professional may seek regular diagnostic information regarding one or more conditions. Biomarkers on cells from a biological sample from a subject, the number of disease cells in a biological sample from a subject, or a particular cell type in a biological from a subject, or cell response to a therapeutic from a biological from a subject can be used with the method and devices provide herein for proving a health-care professional with information to help determine the severity of a disease, predicting the response of the diseased patient to a particular therapeutic drug, or help determine the diagnosis of a disease.

It one aspect the disclosure provides a methods and devices for determining the severity of a disease and providing that information to a health-care professional. It another aspect the disclosure provides a methods and devices for determining or predicting the response a diseased patient to a particular therapeutic drug and providing that information to a health-care professional. It another aspect the disclosure provides a methods and devices for recommending which therapeutic options should be used in the treatment of a disease to a health-care professional. It another aspect the disclosure provides a methods and devices for helping determine the prognosis of a disease to a health-care professional. It another aspect the disclosure provides a methods and devices for helping determine the diagnosis of a disease to a health-care professional.

It is contemplated that the information would be disseminated to a health-care professional by means of a report for clinical management of the patient. The report could be in the form of a electronic form, paper form, or both. The report can also be used with other clinical information from the patient to determine clinical management of the patient. The other clinical information used with the information and data provide by the methods and devices of present disclosure will depend on the disease being treated.

In some applications the devices and methods alone or in combination with other clinical information provide at least 5%, 10%, 15%, 20%, 25%, 30%, 35%, 40%, 45%, 50%, 55% 60%, 65%, 70% ,75%, 80,%, 85%, 90%, or 95% predictive accuracy for drug responsiveness. In some applications the devices and methods alone or in combination with other clinical information provide at least 5%, 10%, 15%, 20%, 25%, 30%, 35%, 40%, 45%, 50%, 55%, 60%, 65%, 70% ,75%, 80,%, 85%, 90%, or 95% predictive accuracy for drug resistance. In some applications the devices and methods alone or in combination with other clinical information provide at least 5%, 10%, 15%, 20%, 25%, 30%, 35%, 40%, 45%, 50%, 55% 60%, 65%, 70% ,75%, 80,%, 85%, 90%, or 95% predictive accuracy for disease prognosis. In some applications the devices and methods alone or in combination with other clinical information provide at least 5%, 10%, 15%, 20%, 25%, 30%, 35%, 40%, 45%, 50%, 55% 60%, 65%, 70% ,75%, 80,%, 85%, 90%, or 95% predictive accuracy for disease diagnosis.

### I. Bacterial Infection Methods

It is contemplated that the methods and devices of the disclosure can be used to detect and pathogenic bacteria or intracellular parasites. Examples of pathogenic bacteria that can be used with the methods and devices of the present disclosure include, but are not limited to, tuberculosis, pneumonia, tetanus, typhoid fever, diphtheria, syphilis, leprosy, or combinations thereof or various species from the genera: *Mycobacterium, Streptococcus, Staphylococcus, Pseudomonas, Shigella, Campylobacter, Burkholderia,* and *Salmonella.* Examples of intracellular parasites include, but are not limited to: *Chlamydophila, Ehrlichia, Mycobacterium, Brucella, Francisella, Legionella, Listeria, Rickettsia,* or combinations thereof.

It one aspect the disclosure provides devices and methods for determining the response to an anti-microbial therapeutic agent in a subject. It one aspect the disclosure provides devices and methods for enumeration of parasites or bacterial cells in a subject. It one aspect the disclosure provides devices and methods for determine the genra or species of parasite or bacterial cell in a subject. It one aspect the disclosure provides devices and methods for the characterization of parasites or bacterial cells, such as intracellular and extracellular markers expressed by a parasite, bacterial cell or viral particle.

It one aspect the disclosure provides devices and methods for determining a bacterial cells in a subject before the presentation of clinical symptoms in said subject. It one aspect the disclosure provides devices and methods for monitoring the efficacy of an anti-microbial therapeutic agent after treatment or in real time. It one aspect the disclosure provides devices and methods for determining the clinical management of a subject suffering from a bacteria, parasite, or viral infection.

### J. Cell Enrichment Methods

For certain applications, it may be advantageous to enrich for a certain cell populations, especially rare cell populations, prior to using the device of the disclosure. Cells can, for example, be selected based on density-based centrifugation, location within a solid tissue, or based on features detectable by microscopic observation, such as location, morphology, or reporter gene expression.

In some applications cells from a bodily fluid or biological specimen can be separated from other components of the bodily fluid or biological specimen. In the instance where the bodily fluid is blood, the blood components may be separated using a Ficoll reagent in conjunction with density-based centrifugation.

Laser cell microdissection (LCM) can be used for separating and isolating specific cell types from complex tissues. In LCM, a laser is used to cause adherence of specific cells to an adhesive backing which is then removed with the cells intact for downstream analysis. It is contemplated that LCM could be used in conjunction with the methods and devices of the disclosure.

Cell fractionation columns may also be used to enrich for a particular cells of interest. For example, cell affinity chromatography can be used to enrich for a cell type of interest. Cells derived from a complex, heterogeneous sample may be separated and isolated by cell affinity chromatography. It is contemplated that affinity chromatography could be used in conjunction with the methods and devices of the disclosure.

Activated cell sorting mechanism, such as flow cytometry, can also be used to enrich or sort specific cell types. Some examples of activated cell sorting mechanisms include, fluorescence activated cell sorting (FACS) and magnetic activated cell sorting (MACS). It is contemplated that FACS or MACS could be used in conjunction with the methods and devices of the disclosure.

Cells derived from a complex biological sample may be separated by other methods known in the art e.g. based on size, cell markers, by using other cell separating devices such micromanipulation, antibody labeled beads, magnets, or the use of semi-automated cell pickers (*e.g.* the Quixell™ cell transfer system from Stoelting Co.). It is contemplated that any of the above-mentioned methods could be used in conjunction with the methods and devices of the disclosure.

The sample to be used with the methods and devices of the present disclosure can comprise a homogenous mixture of cells and/or particles. In other instances, the sample can comprise a heterogeneous mixture of cells and/or particles. The heterogeneous mixture can comprise a plurality of distinct cell types, cell populations, or subpopulations of cells. For example, biological samples may be enriched by increasing the relative cells of interest by at least 10%, 25%, 50%, 60%, 70%, 80%, 90%, 100% or by a factor of at least 1,000, 10,000, 100,000, 1,000,000, 10,000,000, or 100,000,000 of any of the above mentioned sample types.

In some applications, cells can be selected for or enrich by the use of a filtration device that allows for the selection of cells base on cell size, distinguishing cell features (*e.g*. cell surface markers, glycosylation, antigens, ect.). Non-limiting examples of a filtration device that can be used with the methods and devices provide herein are **FIG. 9A, 9B, 9C****,** **10A,** and **10B****.** Other known filtration device such as antibody-based magnetic bead separation can also be used with the methods and devices provided herein.

Any of the devices described herein can further comprise a transition region between a first region and a second region wherein the transition region can comprise obstacles of different sizes for some examples see **(****FIG. 10A** and **FIG. 10B**).

Furthermore, conventional molecular and biochemical techniques can be used in the devices and methods described herein.

### VI. TISSUES, CELLS, AND PARTICLES

In some applications, the methods and devices described herein can be used to analyze single cells, (*e.g*., individual cells). In some applications, the methods and devices can be used to analyze one or more cells. In some applications, the methods and devices can be used to analyze the proliferation of a single cell into a colony of multiple clonal cells. In some applications, the methods and devices can be used to analyze a whole intact biopsy. In some applications, the methods and devices can be used to analyze a single cell suspension from a dissociated biopsy. In some applications, the methods and devices can be used to analyze a tumor *in vivo.* In some applications, the methods and devices can be used to analyze an intact needle biopsy. In some applications, the methods and devices can be used to analyze a homogenous population of cells. In some applications, the methods and devices can be used to analyze a heterogeneous population of cells.

Frequently, the biological sample will be a "clinical sample", which is a sample derived from a patient or subject. Without limitation a clinical sample can be a primary cell line, an immortalized cell line established from a primary cell line, bodily fluids such as blood, frozen tissue, formalin-fixed tissue, paraffin embedded (FFPE) tissue, dissociated tumor specimens, undissociated tumor specimens, and combinations thereof. Clinical samples are advantageous to use with the methods and devices of the disclosure because they provide a rich source of information regarding the various states of gene expression, copy number, and mutations present in a disease cell or at a particular disease stage which can be further used in drug and diagnostic development or clinical management.

In general, clinical samples that can be used include, but are not limited to, sputum, blood, tissue or fine needle biopsy samples, urine, peritoneal fluid, tumor samples, and pleural fluid, or cells therefrom. FFPE samples are a particularly important source for study of archived tissue as they can nucleic acids can be recovered from these samples even after long term storage of the samples at room temperature. See, for example, Specht et al. Am J. Path. (2001), 158(2):419-429. Fresh-frozen biological samples can be used. In some applications, the biological sample to be analyzed is a primary biological sample, which is freshly isolated from a subject or freshly frozen. In other applications cells or organelles to be analyzed can be from non-primary cells such as immortalized cell lines or serially cultured cells derived from a primary biopsy, blood, or other tissue sample.

The cultured cells can be of any type. The cultured cells can be patient cultured cells. The cultured cells can be of known genotype and phenotype or origin. Cultured cells can be mixed with a biological sample to be used as an internal assay control. In some applications, the control cultured cells can be characterized immortalized cell lines. The control cultured cells can be transgenic, that is transformed with one or more genes or with a deletion in one or more genes. In some applications the control cultured cells are known to express certain cellular components or pathways. The control cultured cells can be of known genotype, phenotype, or origin.

The biological sample can be from a subject (*e.g.,* a plant, fungi, eubacteria, archeabacteria, protest, or animal). Frequently, biological samples are samples from a human subject. The subject may be an organism, either a single-celled or multi-cellular organism. The animal can be a mammal. The mammal can be a human, a dog, cat, horse, cow, mouse, rat, chimpanzee, orangutan, gorilla or pig.

Biological samples can include bodily fluids. Bodily fluid generally refers to fluids or secretions originating from a subject. Bodily fluids can comprise a complex and heterogeneous cell population, and may contain cell types such as circulating tumor cells, cancer stem cells, stem cells, fetal cells, bacterial cells, fungal cells. In some applications, bodily fluids can be a mixture of more than one type of bodily fluid. In some applications, bodily fluids can primarily from one type of bodily fluid. Some non-limiting examples of bodily fluid types are blood, urine, semen, vaginal secretions, saliva, amniotic fluid, synovial fluid, bone marrow, spinal fluid, pleural fluid, lymphatic fluid, amniotic fluid, ascites, sputum, or combinations thereof.

The sample may be mixed with one or more samples or cells from the same subject or a different subject. In some applications, a known amount of marked cells may be added to the sample; the marked cells can act as a control to determine the effectiveness of adhesion, the cellular viability, cellular or drug response or combinations thereof.

The cells from the biological sample may be marked by any method known by those skilled in the art. Cells can be marked by prior to using the methods or devices. Cells can be marked by after the use of the methods or devices. Non-limiting example of reagents used to mark cells in a biological sample, include but are not limited to, dyes, fluorescent markers, expression of fluorescent proteins (*e.g*., green fluorescent protein (GFP), red fluorescent protein (RFP), yellow fluorescent protein (YFP), and etc.), expression of bioluminescent proteins (*e.g*. luciferase), expression of tagged proteins; use of radioactive molecules, biotin, horseradish peroxidase, fluororescently-conjugated dextran, β-galactosidase, or genetic alterations to a cell line or an animal (*e.g*. cre-lox recombinase system, FLP recombinase, etc.).

Frequently, the biological sample to be used with the methods and devices of the disclosure are particles derived from a biological sample. Non-limiting examples of particles derived from a biological sample are organelles for example nuclei, mitochondria, endoplasmic reticulum, lysosomes, vesicles, and plastids (*e.g*. chloroplasts). Organelles can be prepared from a biological sample prior to using the device of the disclosure. Organelles can be isolated from a biological sample using the filtration device provided by the present disclosure. In some applications, the biological sample can be a virus particle. In other applications, particles can be man-made non-cellular particles.

The target single cell can be a cancer cell. The target cancer cell can be a cell from any type of cancer, such as an epithelial cancer or blood cancer. Some non-limiting examples of cancers that can be used with the methods and devices include: breast cancer, prostate cancer, colorectal cancer, lung cancer, pancreatic cancer, ovarian cancer, bladder cancer endometrial or uterine cancer, cervical cancer, liver cancer, renal or kidney cancer, thyroid cancer, bone cancer, lymphoma (*e.g*. Hodgkin's lymphoma, non-Hodgkin's lymphoma), circulating tumor, cancer stem cell, melanoma, and non-melanoma skin cancer gliomas, astrocytomas medulloblastomas, choroids plexus carcinomas, ependymomas, brain tumors, neuroblastomas, head and neck cancers, sarcomas, osteosarcomas, rhabdomyosarcomas, Ewing's sarcoma, thyroid cancers, anal cancers, colorectal cancers, endometrial cancers, germ cell tumors, laryngeal cancers, multiple myelomas, prostate cancers, retinoblastomas, gastric cancers, testicular cancers, Wilm's tumor, and normal or healthy biopsy derived from any tissue or organ type.

In some aspects of the disclosure biological tissue samples can be processed into a single cell suspension. A single cell suspension can be obtained from dissociating any tissue type using standard methods known in the art including, for example, by enzymatic digestion with a suitable protease, *e.g*. collagenase, dispase, etc. and the like or by mechanically separating cells in a biological sample.

In some applications, the target cell is rare and may be present at a low ratio in the biological sample. Examples of rare target cells include circulating tumor cells (CTCs), circulating stem cells, fetal stem cells, undifferentiated stem cells, fetal cells, bone marrow cells, progenitor cells, epithelial progenitor cells, endothelial progenitor cells (EPCs), endometrial cells, hematopoietic stem cells (HSCs), circulating tumor cells (CTCs), cancer stem cells (CSCs), and cells that are indicators of early stage of a disease state for example such as early stage cancer, early stage infections for example, viral, bacterial, or fungal infections.

A cell captured and target by the methods and devices provided herein can be any cell type. For example, a target cell can be alive or viable. In other instances, a target cell can be non-viable or dead. A cell may be eukaryote, prokaryote, or from the archaea domain. The cell may be synthetically made or from synthetically made organisms.

Examples of eukaryotic cells that can be used with the disclosure include, but are not limited to, animals, plants, fungi, amoebae, chromalveolata, rhizaria, or excavata cells. Cells from animals can be from: humans, laboratory animals such as mice, rats, monkeys, and chimpanzees; domestic animals such as dogs and cats, agricultural animals such as cows, horses, pigs, sheep, goats; and wild animals such as bears, pandas, lions, tigers, leopards, elephants, zebras, giraffes, gorillas, dolphins, fish, reptiles, birds, and whales.

Examples of prokaryotic cells that can be used with the disclosure can include, but are not limited to, gram-negative, gram-positive bacteria, ungrouped bacteria. The bacteria can of the following: Actinobacteria, Firmicutes, Tenericutes, Aquifacae, Deinococcus-Thermus, Fibrobacteres-Chlorobi/Bacteroidetes, Fusobacteria, Gemmatimonadetes, Nitrospirae, Planctomycetes-Verrucomicrobia/Chlamydiae, Proteobacteria, Spirochaetes, Synergistetes, Acidobacteria, Chloroflexi, Chrysiogenetes, Cyanobacteria, Deferribacteres, Dictyoglomi, Thermodesulfobacteria, Thermotogae, *E. coli, B. subtilis, N. meningitidis, N. gonorrhoeae, S. pneumoniae, S. mutans, S. agalactiae, S. pyogenes, S. aureus, P. aeruginosa, H. pylori, M. catarrhalis, H. influenzae, B. pertussis,* or *C*. *diphtheria.*

### VII. SYSTEMS

### A. Processor and Software

Another aspect of the disclosure provides a system that is configured to implement the methods of the claimed disclosure **(****FIG.13****).**

The system can include a computer server ("server") that is programmed to implement the methods described herein. **FIG. 13** depicts a system **1300** adapted to enable a user to detect, analyze, and process images of cells on the array. The system **1300** includes a central computer server **1301** that is programmed to implement exemplary methods described herein.

The server **1301** includes a central processing unit (CPU, also "processor") **1305** which can be a single core processor, a multi core processor, or plurality of processors for parallel processing. The server **1301** also includes memory **1310** (*e.g.* random access memory, read-only memory, flash memory), electronic storage unit **1315** *(e.g.* hard disk), communications interface **1320** (*e.g*. network adaptor) for communicating with one or more other systems, and peripheral devices **1325** which may include cache, other memory, data storage, and/or electronic display adaptors. The memory **1310,** storage unit **1315,** interface **1320,** and peripheral devices, such as laboratory instrumentation **1325** are in communication with the processor **1305** through a communications bus (solid lines), such as a motherboard. The storage unit **1315** can be a data storage unit for storing data. The server **1301** is operatively coupled to a computer network ("network") **1330** with the aid of the communications interface **1320.** The network **1330** can be the Internet, an intranet and/or an extranet, an intranet and/or extranet that is in communication with the Internet, a telecommunication or data network. The network **1330** in some cases, with the aid of the server **1301,** can implement a peer-to-peer network, which may enable devices coupled to the server **1301** to behave as a client or a server. An optical assay device can be peripheral devices **1325** or remote computer systems **1340.**

The storage unit **1315** can store files, such as individual images, time lapse images, data about individual cells, cell colonies, or any aspect of data associated with the disclosure. The data storage unit **1315** may be coupled with data relating to locations of cells in a virtual grid.

The server can communicate with one or more remote computer systems through the network **1330.** The one or more remote computer systems may be, for example, personal computers, laptops, tablets, telephones, Smart phones, or personal digital assistants.

In some applications the system **1300** includes a single server **1301.** In other situations, the system includes multiple servers in communication with one another through an intranet, extranet and/or the Internet.

The server **1301** can be adapted to store single cell profile information, such as, for example, morphology, shape, cell signaling responses, migratory ability such as epithelial-mesenchymal transition (EMT) status, proliferative capacity, cell death response, kinetic properties, drug response, and/or other information of potential relevance such as drug concentration, time of release, time of response can be collected and stored in a organized database. Such information can be stored on the storage unit **1315** or the server **1301** and such data can be transmitted through a network.

Methods as described herein can be implemented by way of machine (or computer processor) executable code (or software) stored on an electronic storage location of the server **1301,** such as, for example, on the memory **1310,** or electronic storage unit **1315.** During use, the code can be executed by the processor **1305.** In some cases, the code can be retrieved from the storage unit **1315** and stored on the memory **1310** for ready access by the processor **1305.** In some situations, the electronic storage unit **1315** can be precluded, and machine-executable instructions are stored on memory **1310.** Alternatively, the code can be executed on a second computer system **1340.**

Aspects of the systems and methods provided herein, such as the server **1301,** can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such memory (*e.g.*, read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming.

All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and electromagnetic waves, such as used across physical interfaces between local devices, through wired and optical landline networks and over various air-links. The physical elements that carry such waves, such as wired or wireless likes, optical links, or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, tangible storage medium, a carrier wave medium, or physical transmission medium. Non-volatile storage media can include, for example, optical or magnetic disks, such as any of the storage devices in any computer(s) or the like, such may be used to implement the system. Tangible transmission media can include: coaxial cables, copper wires, and fiber optics (including the wires that comprise a bus within a computer system). Carrier-wave transmission media may take the form of electric or electromagnetic signals, or acoustic or light waves such as those generated during radio frequency and infrared data communications. Common forms of computer-readable media therefore include, for example: a floppy disk, a flexible disk, hard disk, magnetic tape, any other magnetic medium, a CD-ROM, DVD, DVD-ROM, any other optical medium, punch cards, paper tame, any other physical storage medium with patterns of holes, a RAM, a ROM, a PROM and EPROM, a FLASH-EPROM, any other memory chip or cartridge, a carrier wave transporting data or instructions, cables, or links transporting such carrier wave, or any other medium from which a computer may read programming code and/or data. Many of these forms of computer readable media may be involved in carrying one or more sequences of one or more instructions to a processor for execution.

The data collected from cell analysis of the system described herein can be presented to a user with the aid of a user interface, such as a graphical user interface on one or more computer devices.

### B. Automation

It is contemplated that methods with the device may be completely or partially automated robotic system. Flexible hardware and software allow instrument adaptability for multiple applications. Thus, any of the methods provide herein can be performed by a computer program product that comprises a computer executable logic that is recorded on a computer readable medium in conjunction with robotics and laboratory equipment.

The software program modules allow creation, modification, and running of methods of the robotic system. Diagnostic modules in the software program allow instrument alignment, correct connections, and motor operations for customized steps of laboratory instruments such as, liquid, particle, cell and organism transfer patterns allow different applications to be performed. Databases allow method and parameter storage. Robotic and computer interfaces allow communication between instruments.

Examples of robotic systems that may be used with the disclosure include, but are not limited to, automated liquid, particle, cell, and organism manipulations such as aspiration, dispensing, mixing, diluting, washing, accurate volumetric transfers, retrieving, and discarding of pipet tips, and repetitive pipetting of identical volumes for multiple deliveries from a single sample aspiration. These manipulations are cross-contamination-free liquid, particle, cell, and organism transfers. This instrument performs automated replication of microplate samples to filters, membranes, and/or daughter plates, high-density transfers, full-plate serial dilutions, and high capacity operation.

It one aspect the disclosure, the robotic system instrumentation will include an optical-based imaging detector. In some applications, useful optical-based imaging can include a microscope(s) with multiple channels of fluorescence, plate readers to provide fluorescent, ultraviolet and visible spectrophotometric detection with single and dual wavelength endpoint and kinetics capability, fluorescence resonance energy transfer, luminescence, quenching, two-photon excitation, and intensity redistribution, CCD cameras to capture and transform data and images into quantifiable formats, and a computer workstation.

It another aspect the disclosure, the robotic system can further include a central processing unit which communicates with a memory and a set of input/output devices (e.g., keyboard, mouse, monitor, printer, etc.) through a bus. The computer executable logic can work in any computer that may be any of a variety of types of general-purpose computers such as a personal computer, network server, workstation, or other computer platform.

It another aspect the disclosure, the automation and robotic systems can include a cell counter such as a Coulter counter, or other cell detector, may also be an integral part of a device rather than constituting a separate device. The counter may utilize any cellular characteristic, e.g., impedance, light absorption, light scattering, or capacitance. In other applications, a cell counter such as a Coulter counter, or other cell detector, is fluidically coupled to an outlet of a device of the disclosure, and a cellular sample is introduced to the device of the disclosure. Cells flowing through the outlet fluidically coupled to the Coulter counter then pass through the Coulter aperture, which includes two electrodes separated by an opening through which the cells pass, and which measures the volume displaced as each cell passes through the opening. Preferably, the Coulter counter determines the number of cells of the enriched sample. Alternatively, the Coulter counter preferably determines the number of cells of diameter of a pre-determined threshold for a particular cell type of interest in the enriched or non-enriched sample.

In some applications of the methods, platforms for multi-well plates, multi-tubes, holders, cartridges, minitubes, deep-well plates, microfuge tubes, cryovials, square well plates, filters, chips, optic fibers, beads, and other solid-phase matrices or platform with various volumes are accommodated on an upgradable modular platform for additional capacity. This modular platform includes a variable speed orbital shaker, and multi-position work decks for source samples, sample and reagent dilution, assay plates, sample and reagent reservoirs, pipette tips, and an active wash station.

### C. Optical-Based Imaging

In some applications, the methods described herein include the use of optical-based imaging. Suitable optical-based imaging instruments to be used with the methods and devices of the disclosure, include but are not limited to, atomic force microscope, scanning tunneling microscopes, electron microscopy, scanning microscopy, mass spectrometers, fluorescence microscopes, flow cytometers, Raman spectrometers, infra-red spectrometers, UV spectrometers, electronic systems, electrochemical systems, optical systems, magnetic and electromagnetic systems, and mass measuring systems and plate readers.

Data collection of electronic and digital imaging can be managed by an appropriately programmed processor. The computer also can transform the data collected during the assay into another format for presentation.

In general, any means of generating a cell count is useful in the methods of the disclosure. Such means include optical, such as scattering, absorption, or fluorescence means. Alternatively, non-aperture electrical means, such as determining capacitance, can also be used and maybe preferable depending on the particular application.

### VIII. METHODS OF MANUFACTURE

The present disclosure also provides for automated assembly of the devices disclosed herein. Various formats that can be used to make a device are illustrated in FIG. **3****.**

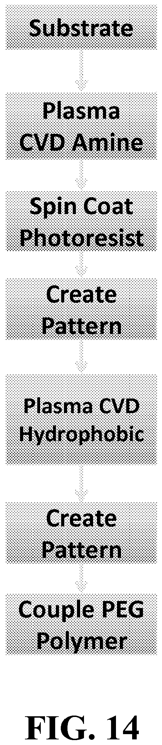

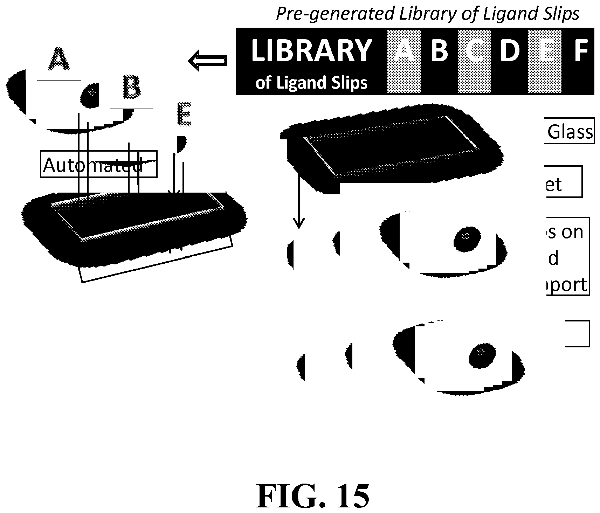

The present disclosure provides a work flow for making 3-D live cell capture arrays **FIG. 14****.** In some applications, the methods of automated assembly of a device (*e.g.* cell capture, agent transfer or filtration device) involving the production and assembly of pre-generated library of ligand slips a gasket or cover glass, and solid support (**FIG. 15**). In other applications, the methods of automated assembly do not include a solid support. Other methods of manufacture of the device are provided in U.S Application No. 61/705914.

The present disclosure provides for a method of making a live cell capture array comprising depositing a hydrogel in an arrayed format onto a solid substrate, wherein said hydrogel comprises a crosslinkable agent; crosslinking said hydrogel to said substrate; contacting said cross-linked hydrogel with a moiety that binds a live target cell and an inducible agent under conditions effective to bind said moiety and said inducible agent to said hydrogel, thereby making a live cell capture surface on said solid substrate. In some applications the live cell capture array will be made without a solid substrate. In some applications the live cell capture surface will be in 2-D (**FIG.1**). In other applications the live cell capture surface will be in 3-D (**FIG. 2** and **FIG.4**). One or more cell-philic sites or domains may be attached, printed, connected, or slipped onto a solid support.

### IX. KITS

It one aspect the disclosure provides kits to produced using the methods and devices provided herein. Kits described herein can be provided, marketed and/or promoted to health care providers, including physicians, nurses, pharmacists, formulary officials, and the like. Kits can also, be marketed directly to the consumer.

Kits will often comprise insert materials, reagents, device components, and instructions on how to perform the methods or test on a particular biological sample type.

A kit will often contain pre-generate library slips loaded with one or more cell binding moiety or stimuli (*e.g*. therapeutic agents, anti-microbial reagents, ect.). The ancillary agents such as buffering agents, stabilizing agents, blocking agents, staining agents, releasing agents, polymerization agents, and the like will often be included in the kit. The kit can be packaged in any suitable manner, typically with all elements in a single container along with a sheet of printed instructions for carrying out the method or test.

The kits can further comprise reagents to enable the detection of cell markers by downstream methods such as RT-PCR, digital droplet PCR, DNA and RNA sequencing, mass spectrometry, immunohistochemistry (IHC), laser cell microdissection (LCM), high content cell screening, flow cytometry, which are suitable for enhancing the information from the mwthods and devices for further clinical detection, prognosis, drug response determination, and diagnosis of a patient suffering from a disease.

In other applications, a kit can further comprise a software package for data analysis of cell profiling, which can include reference profiles for comparison with the patient profile. In some applications the kits software package including connection to a central server to conduct for data analysis and where a report with recommendation on disease state, management ect. can be retrieved by the clinician.

In some applications, the kits can further comprise information, such as scientific literature references, package insert materials, clinical trial results, or summaries of these and the like, which indicate or establish the activities and/or advantages of the composition, and/or which describe dosing, administration, side effects, drug interactions, or other information useful to a health care provider. Such information can be based on the results of various studies, for example, studies using experimental animals involving *in vivo* models and studies based on human clinical trials.

### X. EXAMPLES

### EXAMPLE 1: ISOLATION AND MAINTENANCE OF INDIVIDUAL LIVE CELLS ON A CELL CAPTURE ARRAY DEVICE (PROPHETIC)

The purpose of this study is to isolate single live cells and confirm that human cells can be maintained in a viable state over a five day period on the hydrogel capture sites of a cell capture array.

Cells are grown in flasks and then trypsinize to remove the cells from the flask. The single cell suspension are spun down cells by centrifugation and cells are re-suspended cells in 1.0 mL media. Next, the single cell suspension is dispensed onto the cell capture array device at inlet and spread over the cell-philic surfaces of the device. The array is incubated under standard cell culturing conditions (37 °C, 5% CO₂) for a five day period.

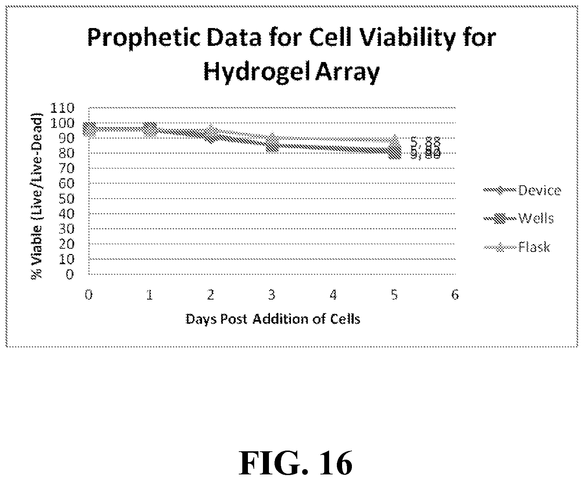

Finally, the captured live single cells are treated with propidium iodide (PI) and the number and presence of live cells on the array is determined over the course of five days. Live cells are indicated the absence of the PI membrane dye by fluorescence microscopy (**FIG. 16**).

### EXAMPLE 2: CAPTURE OF CIRCULATING TUMOUR CELLS FROM BLOOD (PROPHETIC)

The cell-philic surfaces of a cell capture array device (**FIG. 15**) are made with the capture-peptide (*e.g*., cell-adhesive peptide sequence) RGD. Human blood obtained from a subject with cancer is dispensed at inlet, and the blood cells are allows to contact and be bounds to the 3-D cell-philic surface of the device and captured by the RGD peptide (**FIG. 4**). The surface of the device is washed three times with PBS to remove uncaptured cells.

Captured cells are then stained with antibodies against CD45, cytokeratin, EpCAM, vimentin, N-cadherin, and EGFR and visualized by fluorescent microscopy to confirm that CTC cells are bound to the device.

### EXAMPLE 3: DETERMINING A TUMOR CELL'S RESPONSE TO THERAPY (PROPHETIC)

A study with patients suffering from a rare caner a response to response to various anti-cancer therapies can be performed to determine which line of therapy would be most effective in treating the rare cancer.

Three cell capture array devices are pre-loaded with an antibody to EpCAM and 14 different anti-cancer therapeutics at known concentration at known locations on the array (**FIG. 5****,** **FIG. 6A and FIG. 6B**). Next, primary tumor cells are dissociated from the tumor biopsy and dispensed at the inlet of three different arrays and then washed three times in PBS to remove non-target cells (**FIG. 4**). After the tumor cells attach to the cell-philic sites on the array, the array is treated to induce release of the inducible therapeutic agent and to allow it to interact with the captured tumor cell.

After treatment, the captured tumor cells are suspended in the 3-D hydrogel and are stained using standard IHC staining protocols marker expression is detected and measured by fluorescent microscopy for their viability cell death responses to the drugs at 2, 6, and 12 hours after treatment. During the experiment the captured cells on the array are incubated under standard cell culturing conditions (37 °C, 5% CO₂) for the length of the experiment.

After the determined incubation time had occurred, the captured cells are measured for their responses, and a report is generated using a processor and computer-readable media to de-convolute the addressing of treatment types pre-loaded on the cell capture array device with the cellular responses of the captures cells to anti-cancer therapeutics using a system similar to the one shown in **FIG. 13****.**

### EXAMPLE 4: DOSE-DEPENDENT TREATMENT OF INDIVIDUAL LIVE CELLS USING AN AGENT TRANSFER DEVICE (PROPHETIC)

During the drug development, drugs are often screened for their *in vitro* preclinical safety. A dose-dependent drug study can be performed, which allows the amount of a therapeutic agent that causes the therapeutic effect to the amount that causes death or toxicity to be determined.

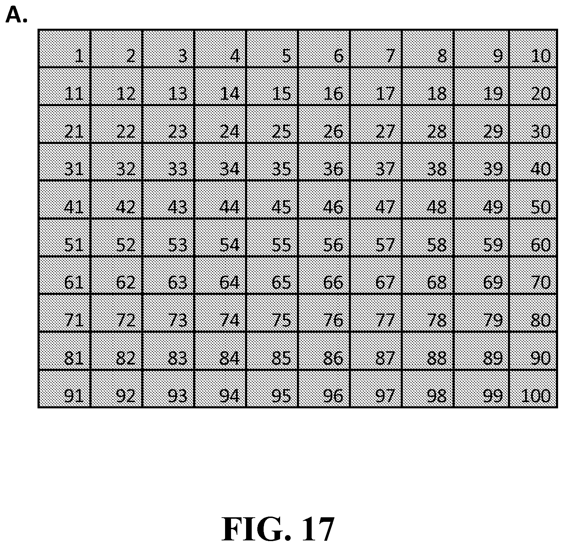

To assess the performance of a cell capture array device in a dose-dependent drug study, a single cell suspension is obtained and dispensed onto a cell capture array device such that the majority of the cell-philic sites on the array are occupied by a single cell (**FIG. 17A**).

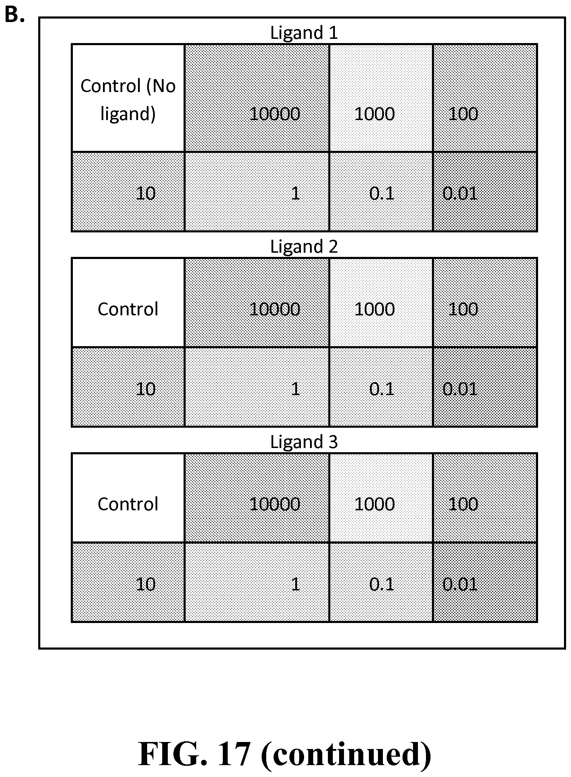

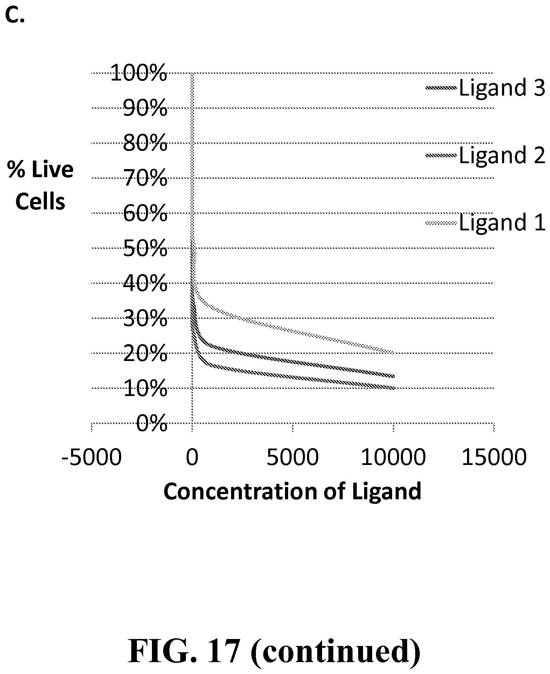

Next, an agent transfer device is pre-loaded with various known amounts of increasing concentrations of ligand (**FIG. 17B**) to study the dose-dependent response of the ligand on individual cells. Finally, cells are assed for their toxicity response to increasing concentrations of the ligand (**FIG. 17C**).

### Protocol:

### STEP 1: Prepare Agent Transfer Device:

- Using acoustic dispensing robot, dispense various concentration of ligands in a hydrogel composition
- Add ligand/hydrogel mixtures to the agent transfer plate in pico to nanoliter volumes.
- Crosslink hydrogel by applying Ultraviolet (UV) light to the array, thereby forming a 3-D hydrogel.

### STEP 2: Prepare Cell Capture Array:

- Grow cells for three days post inoculation
- Trypsinize using standard procedure to generate a single cell suspension
- Spin down cells by centrifugation
- Suspend cells in 1.0 ml RPMI and hydrogel with PI
- Count cells
- Dispense cells on cell capture array device
- Confirm the presence of cell at cell-philic surfaces

### STEP 3: Transfer Agent to Captured Cells:

- Contact the hydrogel surface of the cell capture array device to the hydrogel surface of the agent transfer device, allowing transfer of the ligands harbored on the agent transfer device into the hydrogel of the cell capture array device, wherein they contact the captured live cell.

Perform LIVE/DEAD assay:
- Observe cells on array device by fluorescence microscopy
- Count PI stained and non-PI stained cells

### STEP 4: Perform LIVE/DEAD assay:

- Analyze and plot data

### EXMPLE 5: PRODUCTION OF A CELL FILTRATION DEVICE (PROPHETIC)

Below is a work flow for production of a cell filtration device.

### Protocol:

### STEP 1: Define Channels and Islands

- Create mask
- Pattern slides pre-treated with a hydrophobic surface
- Coat with resist, expose with mask, develop, coat developed surface to be hydrophilic, remove rest of resist

### STEP 2: Define 3-D"posts":

- Pattern islands by the process defined in STEP 1, except to create hydrophilic islands
- Introduce polymeric fluid onto the pre-defined islands by one of several methods: direct dispense to the islands, flow with separation of the fluid specifically onto the islands, dispense through droplets
- Treat polymeric fluid (with Ultraviolet (UV) light) to polymerize to create hydrophobic "posts"
- Treat remaining surface to become hydrophilic

### STEP 3: Define Gasket / Lid Chamber:

- defined by an inlet, an outlet, the body with at least one surface created by a slide fabricated by the processes defined in STEP 1 and STEP 2 above.
- Potentially two opposing surfaces can be defined by the surfaces created by the processes defined above.
- The chamber will include a "loading" zone ahead of the channels and islands to uniformly pressurize the leading face of the chamber.
- Fluid can be introduced into the chamber through the inlet port by means of a syringe or a similar device.
- The fluid, once introduced, will interact with the surfaces that define the chamber as it flows through the chamber and out through the outlet port.

### EXAMPLE 6: FLUID BEHAVIOR ON CELL FILTRATION DEVICE (PROPHETIC)

A cell filtration device that is designed for separating cells and enriching for different sizes in a heterogeneous biological sample can be tested with a water-based fluid to determine whether it is functioning properly.

**Protocol:** Fluid flow behavior on a cell filtration device is assessed a function of:
- Spacing between the top and the bottom surfaces of the chamber (range being investigated (1x of cell dimension to 100x of cell dimension)
- "back pressure" - pressure between the inlet and the outlet. This may be achieved by applying a positive pressure to the inlet or a negative pressure to the outlet (a low vacuum).
- Fluid viscosity: the viscosity of the fluids tested will range between that of water at RTP and that of blood.
- Channel and Island size (50 micron to 1000 microns)
- Channel and Island spacing (50 micron to 1000 microns)

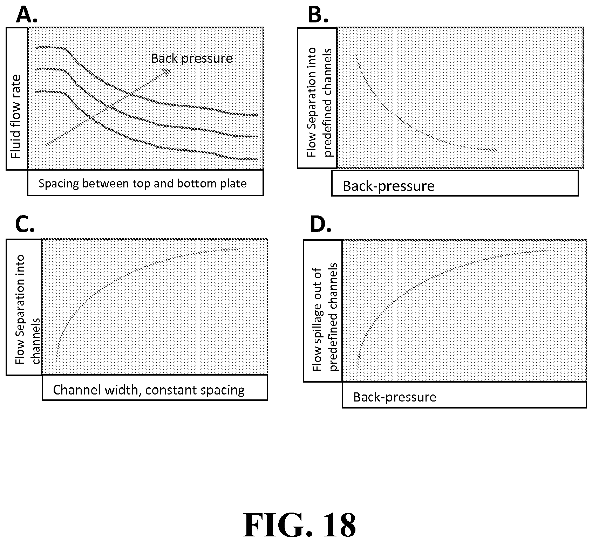

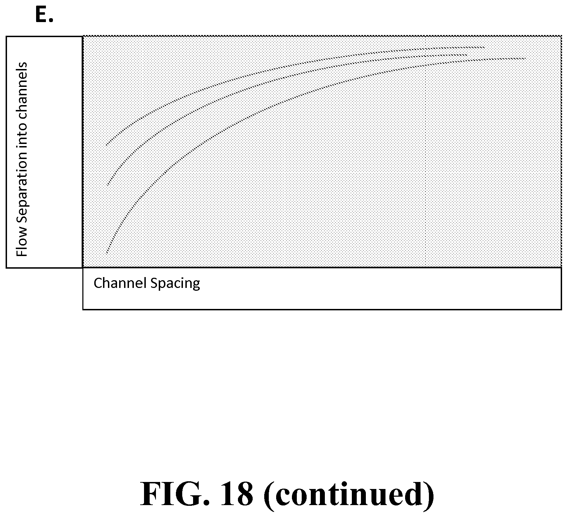

**FIG. 18** illustrates fluid behavior on cell filtration device. **18A** shows fluid flow behavior as a function of between top and bottom plate. **18B** shows back-pressure as a function of flow separation into predefined channels. **18C** shows flow separation into channels as a function of constant spacing. **18D** shows back-pressure as a function of flow spillage out of predefined channels. **18E** show flow separation into channels as a function of channel spacing.

### EXAMPLE 7: PARTICLE SEPARATION ON CELL FILTRATION DEVICE (PROPHETIC)

A cell filtration device that is designed for separating cells and enriching for different sizes in a heterogeneous biological sample is tested with various sized particle to determine whether it is functioning properly.

**Protocol:** Particle separation behavior on a cell filtration device is assessed a function of:
Particle separation, when chamber surfaces are defined by 2-D channels and islands, as a function of:
   - Particle size (10 microns to 400 microns)
   - Channel and Island spacing and size (50 microns to 1000 microns)
Particle separation, when chamber surfaces are defined by 3-D posts, as a function of:
   - Particle size (10 microns to 400 microns)
   - Post size and spacing (50 microns to 1000 microns)

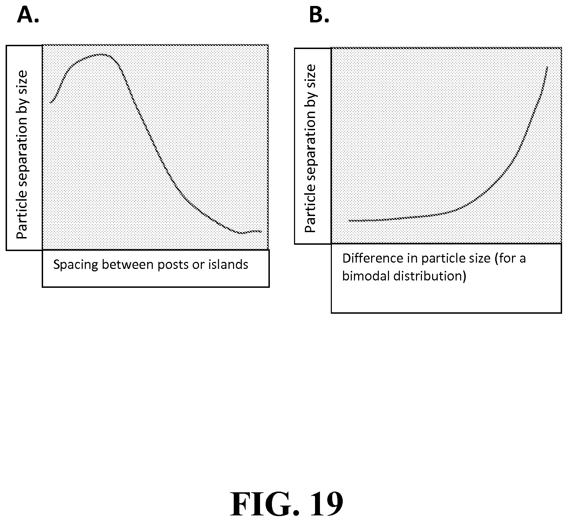

**FIG. 19** illustrates particle separation on cell filtration device. **19A** shows particle separation by size as a function of spacing between posts or islands. **19B** shows particle separation by size as a function of difference in particle size for a bimodal distribution.

While preferred embodiments of the present invention have been shown and described herein, it will be obvious to those skilled in the art that such embodiments are provided by way of example only.

## Claims

1. A method for detecting a response of a live cell comprising:
(a) contacting a live cell to a cell capture array device, wherein said cell capture array device comprises a solid support substrate and a cell capture surface containing a plurality of cell-philic sites arranged thereon;
(b) capturing the live cell in one of said plurality of cell-philic sites;
(c) inducing the release of an inducible-release stimulus into said one of said plurality of cell-philic sites; and
(d) detecting a response of said live cell,
wherein each cell-philic site further comprises a cell capture moiety, and
wherein each cell-philic site is adapted to capture an individual live cell, and
wherein the cell capture array device further comprises a cell phobic coating between the cell-philic sites, and
wherein the cell-philic sites protrude such that they occupy more vertical space than the cell-phobic surface of the cell capture array device.

2. The method of claim 1, wherein in step (a), the device comprises the inducible-release stimulus that is released in step (c).

3. The method of claim 1 or claim 2, wherein the cell capture moiety is selected from the group comprising: a peptide, a protein, a ligand, an antibody, a receptor, a nucleic acid, a glycoprotein, an oligosaccharide, and combinations thereof, or, preferably, an antibody that recognizes a cancer cell.

4. The method of any of claims 1 to 3, wherein said cell-philic site is of a size suitable to accommodate an individual live cell.

5. The method of any of claims 1 to 3, wherein said cell-philic site is of a size suitable to bind a target cell based on the size of said target cell.

6. The method of any of the preceding claims, further comprising:
characterizing at least one of a signaling response of the live cell, cell surface marker expression of the live cell, and DNA expression and sequence of the live cell, and RNA expression and sequence of the live cell.

7. The method of any of the preceding claims, further comprising:
measuring a cellular morphological characteristic of the captured cell.

8. A cell capture device comprising:
a lid gasket comprising an inlet and an outlet connect to a microchannel; wherein said lid gasket is connected to a bottom substrate; and
a bottom substrate comprising a cell capture surface containing a plurality of cell-philic sites arranged thereon,
wherein each cell-philic site further comprises a cell capture moiety, and
wherein each cell-philic site is adapted to capture an individual live cell, and
wherein the cell capture array device further comprises a cell phobic coating between the cell-philic sites, and
wherein the cell-philic sites protrude such that they occupy more vertical space than the cell-phobic surface of the cell capture array device.

9. The device of claim 8, each of said plurality of cell-philic sites comprises an inducible-release stimulus.

10. The device of claim 8 or claim 9, wherein the cell capture moiety is selected from the group comprising: a peptide, a protein, a ligand, an antibody, a receptor, a nucleic acid, a glycoprotein, an oligosaccharide and combinations thereof, or, preferably, an antibody that recognizes a cancer cell.

11. The device of any of claims 8 to 10, wherein said plurality of cell-philic sites are configured in an array format suitable for contact with an agent transfer device.

12. The device of any of claims 8 to 11, wherein the array is on an insertable slip.

## Patentansprüche

1. Verfahren zum Erfassen einer Reaktion einer lebenden Zelle, mit den Schritten:
(a) Kontaktieren einer lebenden Zelle mit einer Zelleinfang-Array-Vorrichtung, wobei die Zelleinfang-Array-Vorrichtung ein festes Trägersubstrat und eine Zelleinfangoberfläche umfasst, die eine Mehrzahl von darauf angeordneten zellphilen Stellen enthält;
(b) Einfangen der lebenden Zelle an einer der Mehrzahl von zellphilen Stellen;
(c) Induzieren der Freisetzung eines induzierbaren Freisetzungsreizes in die eine der Mehrzahl von zellphilen Stellen; und
(d) Erfassen einer Reaktion der lebenden Zelle,
wobei jede zellphile Stelle ferner ein Zelleinfangteil umfasst, und
wobei jede zellphile Stelle eingerichtet ist, um eine einzelne lebende Zelle einzufangen, und
wobei die Zelleinfang-Array-Vorrichtung ferner eine zellphobe Beschichtung zwischen den zellphilen Stellen umfasst, und
wobei die zellphilen Stellen so vorstehen, dass sie mehr vertikalen Raum einnehmen als die zellphobe Oberfläche der Zelleneinfang - Array-Vorrich tung.

2. Verfahren nach Anspruch 1, bei dem in Schritt (a) die Vorrichtung den induzierbaren Freisetzungsreiz umfasst, der in Schritt (c) freigesetzt wird.

3. Verfahren nach Anspruch 1 oder Anspruch 2, bei dem der Zelleinfangteil ausgewählt ist aus der Gruppe umfassend: ein Peptid, ein Protein, einen Liganden, einen Antikörper, einen Rezeptor, eine Nukleinsäure, ein Glykoprotein, ein Oligosaccharid und Kombinationen davon, oder vorzugsweise einen Antikörper, der eine Krebszelle erkennt.

4. Verfahren nach einem der Ansprüche 1 bis 3, wobei die zellphile Stelle eine Größe aufweist, die geeignet ist, eine einzelne lebende Zelle aufzunehmen.

5. Verfahren nach einem der Ansprüche 1 bis 3, wobei die zellphile Stelle eine Größe aufweist, die geeignet ist, eine Zielzelle basierend auf der Größe der Zielzelle zu binden.

6. Verfahren nach einem der vorhergehenden Ansprüche, ferner umfassend:
Charakterisieren wenigstens einer der folgenden: einer Signalantwort der lebenden Zelle, einer Zelloberflächenmarkerexpression der lebenden Zelle, DNA-Expression und -Sequenz der lebenden Zelle und RNA-Expression und -Sequenz der lebenden Zelle.

7. Verfahren nach einem der vorhergehenden Ansprüche, ferner umfassend: Messen einer zellmorphologischen Eigenschaft der eingefangenen Zelle.

8. Zelleinfangvorrichtung, umfassend:
eine Deckeldichtung, die einen Einlass- und einen Auslassanschluss zu einem Mikrokanal umfasst; wobei die Deckeldichtung mit einem Bodensubstrat verbunden ist; und
ein Bodensubstrat, umfassend eine Zelleinfangoberfläche, die eine Mehrzahl von darauf angeordneten zellphilen Stellen enthält,
wobei jede zellphile Stelle ferner ein Zelleinfangteil umfasst, und
wobei jede zellphile Stelle eingerichtet ist, um eine einzelne lebende Zelle einzufangen, und
wobei die Zelleinfang-Array-Vorrichtung ferner eine zellphobe Beschichtung zwischen den zellphilen Stellen umfasst, und
wobei die zellphilen Stellen so vorstehen, dass sie mehr vertikalen Raum einnehmen als die zellphobe Oberfläche der Zelleinfang-Array-Vorrichtung.

9. Vorrichtung nach Anspruch 8, wobei jede der Mehrzahl von zellphilen Stellen einen induzierbaren Freisetzungsreiz umfasst.

10. Vorrichtung nach Anspruch 8 oder Anspruch 9, bei der der Zelleinfangteil ausgewählt ist aus der Gruppe umfassend: ein Peptid, ein Protein, einen Liganden, einen Antikörper, einen Rezeptor, eine Nukleinsäure, ein Glykoprotein, ein Oligosaccharid und Kombinationen davon, oder vorzugsweise einen Antikörper, der eine Krebszelle erkennt.

11. Vorrichtung nach einem der Ansprüche 8 bis 10, wobei die Mehrzahl von zellphilen Stellen in einem Array-Format konfiguriert ist, das für den Kontakt mit einer Wirkstofftransfervorrichtung geeignet ist.

12. Vorrichtung nach einem der Ansprüche 8 bis 11, bei der sich die Anordnung auf einem einlegbaren Streifen befindet.

## Revendications

1. Procédé de détection d'une réponse d'une cellule vivante comprenant les étapes consistant à :
(a) mettre en contact une cellule vivante avec un dispositif de réseau de capture de cellules, où ledit dispositif de réseau de capture de cellules comprend un substrat-support solide et une surface de capture de cellules contenant une pluralité de sites cytophiles agencés sur celle-ci ;
(b) capturer la cellule vivante dans l'un de ladite pluralité de sites cytophiles ;
(c) induire la libération d'un stimulus à libération inductible dans ledit site de ladite pluralité de sites cytophiles ; et
(d) détecter une réponse de ladite cellule vivante, dans lequel chaque site cytophile comprend en outre un fragment de capture de cellules, et
dans lequel chaque site cytophile est adapté pour capturer une cellule vivante individuelle, et
dans lequel le dispositif de réseau de capture de cellules comprend en outre un revêtement cytophobe entre les sites cytophiles, et
dans lequel les sites cytophiles font saillie de sorte qu'ils occupent plus d'espace vertical que la surface cytophobe du dispositif de réseau de capture de cellules.

2. Procédé de la revendication 1, dans lequel, dans l'étape (a), le dispositif comprend le stimulus à libération inductible qui est libéré dans l'étape (c).

3. Procédé de la revendication 1 ou 2, dans lequel le fragment de capture de cellules est choisi dans le groupe comprenant : un peptide, une protéine, un ligand, un anticorps, un récepteur, un acide nucléique, une glycoprotéine, un oligosaccharide et des combinaisons de ceux-ci, ou, de préférence, un anticorps qui reconnaît une cellule cancéreuse.

4. Procédé de l'une des revendications 1 à 3, dans lequel ledit site cytophile a une taille appropriée pour recevoir une cellule vivante individuelle.

5. Procédé de l'une des revendications 1 à 3, dans lequel ledit site cytophile a une taille appropriée pour se lier à une cellule cible sur la base de la taille de ladite cellule cible.

6. Procédé de l'une des revendications précédentes, comprenant en outre l'étape consistant à :
caractériser au moins l'une d'une réponse de signalisation de la cellule vivante, de l'expression de marqueur de surface cellulaire de la cellule vivante, et de l'expression et de la séquence d'ADN de la cellule vivante, et de l'expression et de la séquence d'ARN de la cellule vivante.

7. Procédé de l'une des revendications précédentes, comprenant en outre l'étape consistant à :
mesurer une caractéristique morphologique cellulaire de la cellule capturée.

8. Dispositif de capture de cellules comprenant :
un joint de couvercle comprenant une entrée et une sortie reliées à un microcanal ; où ledit joint de couvercle est relié à un substrat inférieur ; et
un substrat inférieur comprenant une surface de capture de cellules contenant une pluralité de sites cytophiles agencés sur celle-ci,
dans lequel chaque site cytophile comprend en outre un fragment de capture de cellules, et
dans lequel chaque site cytophile est adapté pour capturer une cellule vivante individuelle, et
dans lequel le dispositif de réseau de capture de cellules comprend en outre un revêtement cytophobe entre les sites cytophiles, et
dans lequel les sites cytophiles font saillie de sorte qu'ils occupent plus d'espace vertical que la surface cytophobe du dispositif de réseau de capture de cellules.

9. Dispositif de la revendication 8, chacun de ladite pluralité de sites cytophiles comprend un stimulus à libération inductible.

10. Dispositif de la revendication 8 ou 9, dans lequel le fragment de capture de cellules est choisi dans le groupe comprenant : un peptide, une protéine, un ligand, un anticorps, un récepteur, un acide nucléique, une glycoprotéine, un oligosaccharide et des combinaisons de ceux-ci, ou, de préférence, un anticorps qui reconnaît une cellule cancéreuse.

11. Dispositif de l'une des revendications 8 à 10, dans lequel ladite pluralité de sites cytophiles est configurée dans un format de réseau approprié pour le contact avec un dispositif de transfert d'agent.

12. Dispositif de l'une des revendications 8 à 11, dans lequel le réseau est sur une lamelle pouvant être insérée.
